(19) **Europäisches Patentamt / European Patent Office / Office européen des brevets**

(11) **EP 4 324 444 B1**

(12) **EUROPEAN PATENT SPECIFICATION**

(45) Date of publication and mention
of the grant of the patent:
**24.06.2026 Bulletin 2026/26**

(21) Application number: **23214264.6**

(22) Date of filing: **27.03.2020**

(51) International Patent Classification (IPC):
***A61F 13/537*** *(2006.01)*     ***A61F 13/538*** *(2006.01)*

(52) Cooperative Patent Classification (CPC):
**A61F 13/53708; A61F 13/53747; D04H 1/4258;
D04H 1/43825; D04H 1/43835; D04H 1/4391;
D04H 1/492**

(54) **FLUID MANAGEMENT LAYER FOR AN ABSORBENT ARTICLE**

FLÜSSIGKEITSMANAGEMENTSCHICHT FÜR EINEN SAUGFÄHIGEN ARTIKEL

COUCHE DE GESTION DE FLUIDE POUR UN ARTICLE ABSORBANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.04.2019 US 201962829280 P
11.12.2019 US 201962946725 P
11.12.2019 US 201962946738 P**

(43) Date of publication of application:
**21.02.2024 Bulletin 2024/08**

(62) Document number(s) of the earlier application(s) in
accordance with Art. 76 EPC:
**20720995.8 / 3 946 194**

(73) Proprietor: **The Procter & Gamble Company
Cincinnati, OH 45202 (US)**

(72) Inventors:
• **VIENS, Gerard Alain
Cincinnati, 45202 (US)**
• **NARANJOMARTIN, Carlos Domingo
Toronto, M2N 6K8 (CA)**
• **MELLOR, Paul Dominic
65824 Schwalbach am Taunus (DE)**
• **KNORR, Andreas
65824 Schwalbach am Taunus (DE)**

(74) Representative: **P&G Patent Germany
Procter & Gamble Service GmbH
Sulzbacher Straße 40
65824 Schwalbach am Taunus (DE)**

(56) References cited:
**US-A1- 2014 343 523     US-A1- 2018 098 889**

**Description**

TECHNICAL FIELD

The present disclosure generally relates to fluid management layers for disposable absorbent articles, in particular, fluid management layers that are integrated, carded nonwovens having improved performance characteristics.

BACKGROUND

Disposable absorbent articles such as feminine hygiene products, taped diapers, pant-type diapers and incontinence products are designed to absorb fluids from the wearer's body. Users of such disposable absorbent articles have several desired qualities in their absorbent article of choice. For example, leakage from products like catamenial pads, diapers, sanitary napkins, and incontinence pads is a significant concern. Additionally, comfort and the feel of the product against the wearer's body is also a concern. To provide better comfort, current disposable absorbent articles are typically provided with a topsheet that is flexible, soft feeling, and non-irritating to the wearer's skin. The topsheet is fluid-permeable to allow the fluids to flow into an absorbent core.

Regarding comfort, some consumers may desire a product that has sufficient thickness and stiffness to provide the desirable amount of protection while also being flexible. Lofty materials may be utilized to provide a thick cushiony feeling article. However, in use these lofty materials can experience various compressive loads. Recovery from these compressive loads is paramount in maintaining the cushiony feeling of the article. Exacerbating this issue is the fact that the characteristics of the materials of the absorbent article change once fluid is introduced into the article. Hence, an article that may meet a consumer's requisite criteria before use may no longer be comfortable, flexible, or have the desired stiffness to the user after a given amount of fluid has been absorbed by the absorbent article.

US 2018/0098889 Al relates to disposable absorbent articles, particularly incontinence pads or pants, exhibiting a defined article length, a defined Acquisition Time, a defined dry pad thickness and a particular Energy of Recovery. In some forms, the article comprises a secondary topsheet comprising a homogeneous or heterogeneous mix of absorbent, stiffening and resilient fibers. The resulting spunlaced structure may comprise a plurality of heterogeneous strata which are - after the spunlacing process - integral with one another. US 2014/0343523 Al is concerned with a hydroentangled fibrous structure, which can be incorporated into an absorbent article. The fibrous structure may include cellulosic fibers, non-cellulosic fibers, and polyolefin-based binder fibers. In some forms, the absorbent article comprises a secondary topsheet comprising the hydroentangled fibrous structure having a defined basis weight.

As such there is a need to create fluid management materials that have sufficient caliper and compressive recovery for use in absorbent articles.

SUMMARY

The invention provides a fluid management layer and an absorbent article as defined in the claims.

BRIEF DESCRIPTION OF THE DRAWINGS

While the specification concludes with claims particularly pointing out and distinctly claiming the subject matter that is regarded as the present invention, it is believed that the invention will be more fully understood from the following description taken in conjunction with the accompanying drawings. Some of the figures may have been simplified by the omission of selected elements for the purpose of more clearly showing other elements. Such omissions of elements in some figures are not necessarily indicative of the presence or absence of particular elements in any of the exemplary embodiments, except as may be explicitly delineated in the corresponding written description. None of the drawings are necessarily to scale.

FIG. 1A is a schematic representation of a disposable absorbent article constructed in accordance with the present disclosure;
FIG. 1B is a schematic representation of an absorbent system of the disposable absorbent article shown in FIG. 1A;
FIG. 2 is a schematic representation of a process which can be utilized to construct fluid management layer of the present disclosure;
FIG. 3 is a schematic representation of an elevation view of a fluid management layer constructed in accordance with the present disclosure;
FIG. 4 is graphical plot showing results from a dynamic mechanical analysis test performed on the fluid management layer of the present disclosure versus a comparative sample;
FIGS. 5A-5D are SEM images showing cross sections of a fluid management layer which is a comparative sample;

and

FIGS. 6A-6D are SEM images showing cross sections of a fluid management layer of a fluid management layer constructed in accordance with the present disclosure.

## DETAILED DESCRIPTION

As used herein, the following terms shall have the meaning specified thereafter:
"Absorbent article" refers to wearable devices, which absorb and/or contain liquid, and more specifically, refers to devices, which are placed against or in proximity to the body of the wearer to absorb and contain the various exudates discharged from the body. Absorbent articles can include diapers, training pants, adult incontinence undergarments (e.g., liners, pads and briefs) and/or feminine hygiene products.

The term "integrated" as used herein is used to describe fibers of a nonwoven material which have been intertwined, entangled, and/or pushed / pulled in a positive and/or negative Z-direction (direction of the thickness of the nonwoven material). Some exemplary processes for integrating fibers of a nonwoven web include spunlacing and needlepunching. Spunlacing uses a plurality of high pressure water jets to entangle fibers. Needlepunching involves the use of needles to push and/or pull fibers to entangle them with other fibers in the nonwoven.

The term "carded" as used herein is used to describe structural features of the fluid management layers described herein. A carded nonwoven utilizes fibers which are cut to a specific length, otherwise known as "staple length fibers." Staple length fibers may be any suitable length. For example, staple length fibers may have a length of up to 120 mm or may have a length as short as 10 mm. However, if a particular group of fibers are staple length fibers, for example viscose fibers, then the length of each of the viscose fibers in the carded nonwoven is predominantly the same, i.e. the staple length. It is worth noting that where additional staple fiber length fiber types are included, for example, polypropylene fibers, the length of each of the polypropylene fibers in the carded nonwoven is also predominantly the same. But, the staple length of the viscose and the staple length of the polypropylene may be different.

In contrast, continuous filaments such as by spunbonding or meltblowing processes, do not create staple length fibers. Instead, these filaments are of an indeterminate length and are not cut to a specific length as noted regarding their staple fiber length counterparts.

The "longitudinal" direction is a direction running parallel to the maximum linear dimension, typically the longitudinal axis, of the article and includes directions within 45° of the longitudinal direction. "Length" of the article or component thereof, when used herein, generally refers to the size/distance of the maximum linear dimension, or typically to the size/distance of the longitudinal axis, of an article or part thereof.

The "lateral" or "transverse" direction is orthogonal to the longitudinal direction, i.e. in the same plane of the majority of the article and the longitudinal axis, and the transverse direction is parallel to the transverse axis. "Width" of the article or of a component thereof, when used herein, refers to the size/distance of the dimension orthogonal to the longitudinal direction of the article or component thereof, i.e. orthogonal to the length of the article or component thereof, and typically it refers to the distance/size of the dimension parallel of the transverse axis of the article or component.

The "Z-direction" is orthogonal to both the longitudinal and transverse directions.

"Machine Direction" or "MD" as used herein means the direction parallel to the flow of the carded staple-fiber nonwoven through the nonwoven making machine and/or absorbent article product manufacturing equipment.

"Cross Machine Direction" or "CD" as used herein means the direction parallel to the width of the carded staple-fiber nonwoven making machine and/or absorbent article product manufacturing equipment and perpendicular to the machine direction.

An integrated, carded nonwoven as disclosed herein can be used in a variety of disposable absorbent articles, but is particularly useful in diapers, feminine hygiene products and incontinence products such as sanitary napkins and incontinence pads. The integrated, carded nonwovens of the present disclosure can be particularly effective as a fluid management layer in the above absorbent articles. Additionally, the integrated, carded nonwoven of the present disclosure provides increased caliper, even at lower basis weights, has good compressions recovery characteristics, and has good fluid handling properties.

The fluid management layer of the present disclosure maybe arranged between a topsheet and a backsheet in a disposable absorbent article. An absorbent core may be disposed between the topsheet and the backsheet as well. In such instances, the fluid management layer may be disposed between the topsheet and the absorbent core.

As noted previously, the fluid management layer is an integrated, carded nonwoven material. The fluid management layer of the present disclosure may comprise one or more carded webs which are subsequently fiber integrated with one another. Where only one carded web is utilized, the fibers of the carded web are integrated.

A wide variety of configurations for a fluid management layer may be achieved. However, it is important that the fluid management layer of the present disclosure have adequate openness to allow for quick acquisition of fluid yet also be able to lock away liquid insults to reduce the likelihood of rewet. With this in mind, the carded webs which make up the fluid management layer may be different from one another. For example, one of the carded webs may comprise a different fiber blend than the others. Specifically, assuming the first carded web would be closest to the wearer-facing surface in an absorbent article, the fiber selection for a first carded web may be such that there is more openness associated with this web. A second carded web may be similarly configured. In contrast, a third carded web may be configured to collect liquid insults from the void space of the first and second carded webs and effectively distribute these liquid insults to an absorbent core (heterogeneous configuration). Alternatively, the first carded web, the second carded web and the third carded web may be configured the same (homogeneous configuration).

Once the carded web(s) are integrated, they cannot be manually separated - at least not without substantial effort and time. Each carded nonwoven web forms a stratum in the overall fluid management layer. Each stratum can maintain its unique properties for at least a portion of the stratum along the z-direction, even when integrated into a larger fluid management layer. The fluid management layer can provide capillary suction to "pull" fluid through the topsheet, which is competing for trickle/low flow conditions. The fluid management layer also can contain a gush by providing distribution functions to efficiently utilize the absorbent core, as well as provide intermediate storage until the absorbent core can accept fluid.

As noted previously, absorbent articles which exhibit a soft cushiony feel, good resiliency and fluid handling characteristics is in accordance with the present disclosure. The caliper of the fluid management layer therein is important. Notably, typical calipers of webs from conventional spunlace lines achieve a caliper factor (caliper per 10 gsm of basis weight) of 0.03 to 0.12. In contrast, the fluid management layers of the present disclosure can exhibit a caliper factor of at least 0.13 mm, more preferably at least about 0.15 mm, or most preferably about 0.2 mm, including any values within these ranges and any ranges created thereby. The fluid management layer of the present disclosure can have a caliper factor of between 0.13 mm to about 0.3 mm, or more preferably from about 0.14 mm to about 0.25 mm, or most preferably from about 0.15 mm to about 0.22 mm, including all values within these ranges and any ranges created thereby. Caliper data is provided hereafter for an Inventive Sample as well as a Comparative Sample. The caliper and caliper factor of the fluid management layers of the present disclosure may be determined by the Caliper and Caliper Factor test methods disclosed herein. It is important to note that the caliper factors mentioned heretofore are with regard to caliper obtained using 0.5 kPa of applied pressure as noted in the Caliper method disclosed herein.

The inventors have surprisingly discovered that in order to achieve the increase in caliper factor, a simpler process path may be utilized to produce the spunlace web. Generally, the web path through a hydroentangling line is tortuous and subjects the web to both compressive and tensile stresses. This tortuous web path requires water jet pressures high enough to entangle the fibers, creating tensile strength sufficient to survive subsequent web handling. These water jets are applied to both surfaces of the web. This additional water pressure required to create sufficient entanglement for tensile strength is generally in excess of the pressure needed to create the desired fluid handling pore structure and meaningfully reduces caliper of the resultant web. Additionally, the web is subject to significant radial compression and tensile stress as the web is wound around a variety of vacuum drums and rolls such that additional water jets can further entangle the constituent fibers of the strata. Moreover, these webs may be subsequently wound around dryer drums subjecting them to additional compressive force. However, the inventors have found that winding of the web around these rolls causes compression on the web and actually reduces the caliper of the web.

In contrast, the inventors have discovered that through the use of a simplified web path that reduces radial compressive stresses/excessive tensile forces and the appropriate selection of fibers in the fluid management layer, caliper of the fluid management layers of the present disclosure can be maintained. For example, the use of rolls and the number of water jets utilized can be reduced via the simplified path. As such, while the level of entanglement is not to the extent provided by the conventional process, sufficient tensile strength in the web can be provided by selecting the appropriate combination of fibers as disclosed herein, e.g. stiffening fibers which can be heat treated. Again, the simplified path and appropriate fiber selection as described herein, allows the fluid management layers of the present disclosure to achieve caliper factors that have heretofore not been achievable.

Additionally, the caliper factors of the fluid management layers of the present disclosure mentioned above were derived from caliper data from material which was rolled for storage / shipping. Caliper measures pre-winding could be taken which would yield much higher caliper factors. However, such caliper measurements may not necessarily reflect the fluid management layer that makes it into an absorbent article.

The fluid management layer of the present disclosure can have a basis weight of up to 75 grams per square meter (gsm); or a basis weight of up to 70 gsm; or a basis weight preferably in the range of between about 30 gsm to about 75 gsm, more preferably from about 45 gsm to about 70 gsm, and most preferably between about 50 gsm to about 65 gsm, including any values within these ranges and any ranges created thereby. In one specific example, the fluid management layer of the present disclosure may have a basis weight of about 55 gsm.

Some absorbent articles may not require as much basis weight as recited above. For example, liners which generally do not have the same level of absorbent capacity as menstrual pads may be able to have a reduced basis weight over that which was recited above. For example, the fluid management layer may have a basis weight of between 20 gsm to 70 gsm or more preferably between 35 gsm to about 65 gsm, or most preferably from about 40 gsm to about 60 gsm, specifically including all values within these ranges and any ranges created thereby. In one specific example, the fluid management layer of the present disclosure can have a basis weight of about 40 to about 50 gsm. The basis weight of fluid management layers in accordance with the present disclosure may be determined via the Basis Weight test method disclosed herein.

The inventors have also found that the processing technique for creating caliper in the fluid management layer can be utilized not only on spunlace materials where the strata are heterogeneous but also where the strata are homogeneous, e.g. each stratum has the same fiber makeup. Additionally, the inventors have surprisingly found that spunlace materials constructed with this process along with appropriate fiber selection can also provide good resiliency and recovery from compression, with improved fluid handling performance above those spunlace materials that are produced via typical spunlace processes.

It is also worth noting that due to the fiber integration, the fluid management layer does not require adhesives or latex binders for stability. Additionally, the integrated, carded nonwoven of the fluid management layer can be manufactured from an assortment of suitable fiber types that produce the desired performance characteristics. For example, the fluid management layer may comprise a combination of stiffening fibers, absorbent fibers and resilient fibers.

As will be discussed in additional detail below, the types of fibers in the fluid management layer of the present disclosure are described in terms of their functionality within the fluid management layer. For example, absorbent fibers are utilized to absorb liquid insults. Stiffening fibers are utilized to bond together via heat treatment thereby providing stiffness and resiliency to the fluid management layer. Resilient fibers are utilized to provide recovery from compressive forces which act against the fluid management layer.

In order to enhance the stabilizing effect of the integration, crimped fibers may be utilized. One or more of the absorbent fibers, stiffening fibers, and resilient fibers may be crimped prior to integration. For example, where synthetic fibers are utilized, these fibers may be mechanically crimped via intermeshing teeth. And for the absorbent fibers, these fibers may be mechanically crimped and/or may have a chemically induced crimp due to the variable skin thickness formed during creation of the absorbent fibers.

As noted previously, absorbent fibers can provide absorption of liquid insults from the wearer-facing surface or topsheet. However, when absorbent fibers absorb liquid, they tend to lose some of their structural integrity. The loss of structural integrity can reduce the resiliency of the absorbent article and lead to increased bunching and increased leakage. Accordingly, while in principle, a large percentage of absorbent fibers is good for draining liquid insults from the wearer-facing surface and/or topsheet rapidly, the large percentage will lead to other problems with the absorbent article as mentioned heretofore.

In light of the potential problems associated with having too much of a weight percentage of absorbent fibers, the inventors have found that the fluid management layer of the present disclosure must comprise from about 10 percent to about 60 percent by weight, more preferably from about from about 15 percent to about 50 percent by weight, most preferably from about 20 percent to about 40 percent by weight, specifically including any values within these ranges and any ranges created thereby of absorbent fibers. In one specific example, the fluid management layer may comprise about 30 percent by weight of absorbent fibers. In yet another specific example, the fluid management layer may comprise about 20 percent absorbent fibers. The weight percentages of the absorbent fibers, resilient fibers and/or stiffening fibers may be determined via the Material Compositional Analysis method described herein.

Additionally, due to the loss of integrity of the absorbent fibers when wet, the fluid management layer also may comprise sufficient weight percentage of resilient fibers which impact the recovery of the absorbent article from compressive loads experienced during use. The inventors have found that the fluid management layer of the present disclosure must comprise from about 15 percent to about 70 percent, more preferably from about 20 percent to about 60 percent, or most preferably from about 25 percent to about 50 percent by weight of resilient fibers, specifically reciting all values within these ranges and any ranges created thereby. In one specific example, the fluid management layer may comprise about 40 percent by weight resilient fibers. In another specific example, the fluid management layer may comprise about 30 percent by weight of resilient fibers.

Moreover, stiffening fibers may be utilized to help the fluid management layer of the present disclosure provide resiliency to the absorbent article. For example, as discussed hereafter, stiffening fibers may be bonded to one another via heat treatment of the fluid management layer during production. This bonding of the stiffening fibers creates a support matrix which helps with resiliency and stiffness of the fluid management layer. With this in mind, the fluid management layer comprises from about 25 percent to about 70 percent, more preferably from about 30 percent to about 60 percent, or most preferably from about 40 percent to about 55 percent of stiffening fiber, specifically reciting all values within these ranges and any ranges created thereby. In one specific example, the fluid management layer may comprise about 50 percent by weight of stiffening fibers.

As mentioned previously, the fluid management layers of the present disclosure can provide their respective absorbent articles with a soft cushiony feel with good resiliency. Where caliper, resiliency, and a soft cushiony feel are the objective, the weight percentage of stiffening fibers may be greater than or equal to the weight percentage of resilient fibers. The weight percentage of absorbent fibers can be less than the weight percentage of resilient fibers and/or stiffening fibers. In general, a higher weight percentage of absorbent fibers is considered to be beneficial in rapidly absorbent fluid insults; however, given the proximity of the absorbing fibers to the topsheet, it is beneficial for the absorbent core to dewater the absorbing fibers. Where there is a larger percentage of absorbing fibers, typically a larger core is required to dewater the absorbent fibers. This typically leads to higher costs. With this in mind, a ratio of absorbent fibers in the fluid management layers of the present disclosure to stiffening fibers by weight percentage can be from about 1:7 to about 2:1, more preferably from about 1:4 to about 1.5:1, most preferably from about 1:2 to about 1:1, specifically reciting all values within these ranges and any ranges created thereby Similarly a ratio of absorbent fibers to resilient fibers by weight percentage can be from about 1:7 to about 3:1, more preferably from about 1:2 to about 2:1, or most preferably from about 1:1.5 to about 1:1, specifically reciting all values within these ranges and any ranges created thereby.

Regardless of whether the fluid management layer is utilized in an adult incontinence article menstrual article, liner, or other hygiene article, of importance is the ability of the fluid management layer to acquire liquid insults from the topsheet and to pull the liquid far enough from the topsheet, such that the topsheet does not feel wet. To accomplish this, the inventors have found that the increased caliper of the fluid management layer discussed herein can facilitate fluid acquisition due to the increased void volume of the fluid management layer. The higher caliper at the lower basis weight equals more void volume with higher permeability. Additionally, the increased caliper of the fluid management layer can also provide a stain masking benefit. Namely, the stains that are visible through the topsheet with absorbent articles using the fluid management layer of the present disclosure, appear much smaller than their conventional fluid management layer counterparts.

It is worth noting that for a set basis weight of a fiber, larger diameter fibers can provide more void volume between adjacent fibers as compared to their smaller diameter counterparts. As such, the fiber size of the fibers in the strata closest to the topsheet may be of importance. For example, for a set percentage weight of fiber, as fiber size goes up there are fewer fibers per gram, and fewer fibers can equal more space between the fibers. Ideally, particularly in the context of menstrual fluid, the fluid management layer can have void volume to drain the topsheet.

With the above in mind, the inventors have also surprisingly discovered that careful selection of the fiber types in each of the strata in the fluid management layer and the linear densities of the fiber types can achieve the desired outcome of quick acquisition. The fiber types of the individual strata are discussed in additional detail hereafter. It is worth noting that the discussion below regarding fiber types in the strata of the fluid management layer assumes that the first carded nonwoven web is nearer to the topsheet than the web(s) of the additional card(s).

Some suitable linear density values of absorbent fibers for use in the fluid management layers of the present disclosure are provided. For example, the absorbent fiber linear density may range from about 1 dtex to about 7 dtex, more preferably from about 1.4 dtex to about 6 dtex, or most preferably from about 1.7 dtex to about 5 dtex, specifically reciting all values within these ranges and any ranges created thereby. In one specific example, the absorbent fiber may comprise a linear density of about 1.7 dtex. The decitex (dtex) values for the absorbent fibers, stiffening fibers and/or resilient fibers may be determined via the Fiber Decitex method disclosed herein.

The absorbent fibers of the fluid management layer may have any suitable shape. Some examples include trilobal, "H," "Y," "X," "T," round, or flat ribbon. Further, the absorbing fibers can be solid, hollow or multi-hollow. Other examples of suitable multi-lobed, absorbent fibers for utilization in the fluid management layers detailed herein are disclosed in U.S. Patent No. 6,333,108 to Wilkes et al, U.S. Patent No. 5,634,914 to Wilkes et al., and U.S. Patent No. 5,458,835 to Wilkes et al. The trilobal shape can improve wicking and improve masking. Suitable trilobal rayon is available from Kelheim Fibres and sold under the trade name Galaxy. While each stratum may comprise a different shape of absorbing fiber, much like mentioned above, not all carding equipment may be suited to handle such variation between / among strata. In one specific example, the fluid management layer comprises round absorbent fibers.

Any suitable absorbent material for the absorbent fibers may be utilized. Some examples of absorbent materials include cotton, pulp, rayon or regenerated cellulose or combinations thereof. In one example, the fluid management layer may comprise viscose cellulose fibers. The length of the absorbent fibers can be in the range of about 20 mm to about 100 mm, or more preferably about 30 mm to about 50 mm or most preferably about 35 mm to about 45 mm, specifically reciting all values within these ranges and any ranges created thereby. In general, the fiber length of pulp is from about 4 to 6 mm and cannot used in conventional carding machines because the pulp fibers are too short. So, if pulp is desired as a fiber in the fluid management layer, then additional processing to add pulp to the carded webs may be required. As an example, pulp may be airlaid between carded webs with the combination being subsequently integrated. As another example, tissue may be utilized in combination with the carded webs and the combination may be subsequently integrated.

As noted previously, in addition to absorbent fibers, the fluid management layer of the present disclosure may comprise stiffening fibers. Stiffening fibers may be utilized to help provide structural integrity to the fluid management layer. The stiffening fibers can help increase structural integrity of the fluid management layer in a machine direction and/or in a cross-machine direction which can facilitate web manipulation during processing of the fluid management layer for incorporation into a disposable absorbent article.

Some suitable linear density values of stiffening fiber are provided. For example, the stiffening fiber linear density may range from about 1.0 dtex to about 6 dtex, more preferably from about 1.5 dtex to about 5 dtex, or most preferably from about 2.0 dtex to about 4 dtex, specifically reciting all values within these ranges and any ranges created thereby. In another specific example, the dtex of the stiffening fibers is about 2.2 dtex.

Some examples of suitable stiffening fibers include bi-component fibers comprising polyethylene and polyethylene terephthalate components or polyethylene terephthalate and co-polyethylene terephthalate components. The components of the bi-component fiber may be arranged in a core sheath arrangement, a side by side arrangement, an eccentric core sheath arrangement, a trilobal arrangement, or the like. In one specific example, the stiffening fibers may comprise bi-component fibers having polyethylene / polyethylene terephthalate components arranged in a concentric, core - sheath arrangement where the polyethylene is the sheath.

While other materials may be useful, the inventors have found that the stiffness of polyethylene terephthalate is useful in creating a resilient structure. In contrast, the polyethylene component of the stiffening fibers can be utilized to bond to one another during heat treatment. This can help provide tensile strength to the web in both the MD and CD. Additionally, the bonding of the polyethylene component to other polyethylene components of stiffening fibers can create fixed points in the nonwoven. These fixed points can reduce the amount of fiber-to-fiber sliding which can increase the resiliency of the material.

One of the benefits of the stiffening fibers is that the integrated nonwoven may be heat treated post fiber entanglement. The heat treatment can provide additional structural integrity to the integrated nonwoven by forming bonds between adjacent stiffening fibers. So, where there is a higher percentage of stiffening fibers, more connection points may be created. Too many connection points can yield a much stiffer fluid management layer which may negatively impact comfort. As such, the weight percentage of the stiffening fibers is of critical importance when designing an absorbent article.

Regarding the heat stiffening process, any suitable temperature may be utilized. And, the suitable temperature may be impacted, in part, by the constituent chemistry of the stiffening fibers as well as by the processing fluid management layer web. For example, the fluid management layer web may be heat stiffened at a temperature of 132 degrees Celsius. However, it is also worth noting, that in order to provide a uniform stiffness property across the fluid management layer, any heating operation should be set up to provide uniform heating to the fluid management layer web. Even small variations in temperature can greatly impact the tensile strength of the fluid management layer.

As noted previously, the fluid management layer of the present disclosure comprises resilient fibers. The resilient fibers can help the fluid management layer maintain its permeability and compression recovery. Any suitable size fiber may be utilized. For example, the resilient fibers can have a linear density of about 4 dtex to about 15 dtex, more preferably from about 5 dtex to about 12 dtex, or most preferably from about 6 dtex to about 10 dtex, specifically reciting all values within these ranges and any ranges created thereby. In another specific example, the fluid management layer may comprise resilient fibers having variable cross sections, e.g. round and hollow spiral, and/or may comprise resilient fibers having variable dtex's. In yet another specific example, the resilient fibers of the present disclosure may comprise a dtex of about 10. In such forms, the resilient fibers may be hollow spiral.

The resilient fibers can be any suitable thermoplastic fiber, such as polypropylene (PP), polyethylene terephthalate, or other suitable thermoplastic fibers known in the art. The length of the resilient fibers can be in the range of about 20 mm to about 100 mm, or more preferably about 30 mm to about 50 mm or most preferably about 35 mm to about 45 mm. The thermoplastic fibers can have any suitable structure or shape. For example, the thermoplastic fibers can be round or have other shapes, such as spiral, scalloped oval, trilobal, scalloped ribbon, and so forth. Further, the PP fibers can be solid, hollow or multi-hollow. The resilient fibers may be solid and round in shape. Other suitable examples of resilient fibers include polyester/co-extruded polyester fibers. Additionally, other suitable examples of resilient fibers include bi-component fibers such as polyethylene / polypropylene, polyethylene / polyethylene terephthalate, polypropylene / polyethylene terephthalate. These bi-component fibers may be configured as a sheath and a core. The bi-component fibers may provide a cost-effective way to increase basis weight of the material while additionally enabling optimization of the pore size distribution.

The resilient fibers can be polyethylene terephthalate (PET) fibers, or other suitable non-cellulosic fibers known in the art. The PET fibers can have any suitable structure or shape. For example, the PET fibers can be round or have other shapes, such as spiral, scalloped oval, trilobal, scalloped ribbon, hollow spiral, and so forth. Further, the PET fibers can be solid, hollow or multi-hollow. In one particular example, fibers may be fibers made of hollow/spiral PET. Optionally, the resilient fibers may be spiral-crimped or flat-crimped. The resilient fibers may have a crimp value of between about 4 and about 12 crimps per inch (cpi), or between about 4 and about 8 cpi, or between about 5 and about 7 cpi, or between about 9 and about 10 cpi. Particular non-limiting examples of resilient fibers can be obtained from Wellman, Inc. Ireland under the trade names H1311 and T5974. Other examples of suitable resilient fibers for utilization in the carded staple-fiber nonwovens detailed herein are disclosed in U.S. Patent No. 7,767,598 to Schneider et al.

It is worth noting that the stiffening fibers and resilient fibers should be carefully selected. For example, while the constituent chemistries of the stiffening fibers and the resilient fibers may be similar, resilient fibers should be selected such that their constituent material's melting temperature is higher than that of the stiffening fibers. Otherwise, during heat treatment, resilient fibers would bond to stiffening fibers and vice versa and could create an overly rigid structure.

Without wishing to be bound by theory, it is believed that for weight percentage of absorbent fibers above about 30 percent, within the gsm ranges disclosed herein, the resilient fibers and/or stiffening fibers should be carefully selected. Where a soft, cushiony fluid management layer with a caliper factor of at least 0.13 or greater as described herein, the resilient and/or stiffening fibers can be selected to counteract the loss of structural integrity of the absorbent fibers when wet. For example, a higher dtex of resilient fiber may be beneficial in counteracting the loss of integrity experienced by the absorbent fibers. In such instances, resilient fibers may be utilized having a dtex of between about 5 dtex to about 15 dtex, more preferably from about 6 dtex to about 12 dtex, or most preferably from about 7 dtex to about 10 dtex.

In addition to or an alternative thereof, the stiffening fibers may be configured to provide greater structural integrity. For example, the stiffening fibers may comprise bicomponent fibers in a core-sheath configuration where the sheath is co-polyethylene terephthalate. However, with such a material change, additional problems may occur. For example, the joining of materials to the fluid management layer may then only be via adhesive as opposed to fusion bonding.

Still another example which is in addition to or independent of the foregoing is an increase in bonding of the stiffening fibers. Where the absorbent fibers comprise more than 30 percent by weight of the fluid management layer, the heat at which the stiffening fibers are bonded may be increased and/or the time of exposure may be increased. This can increase the number of bonds in the stiffening fiber matrix which can counteract the loss of integrity of the absorbent fibers when wet. However, with the increase in the number of bonds comes an increase in stiffness. The increase in stiffness can decrease the perception of softness by the user. In a similar regard, in addition to or alternatively thereto, the linear density of the stiffening fibers may be increased to combat the loss of integrity of the absorbent fibers, where the absorbent fibers make up 30 percent by weight or more. In such instances, the linear density of the stiffening fibers may be from about 3 dtex to about 6 dtex, more preferably from about 4 dtex to about 6 dtex.

It is worth noting that while it may appear that the solution to wet collapse is simply to use larger dtex fibers, their use must be balanced. Particularly for viscous fluids, the fluid management layers of the present disclosure can have some degree of capillarity to help drain liquid insults to the wearer-facing surface of the article. Unfortunately, while the use of large dtex fibers can provide caliper benefits, it also detracts from capillarity which can lead to fluid handling issues.

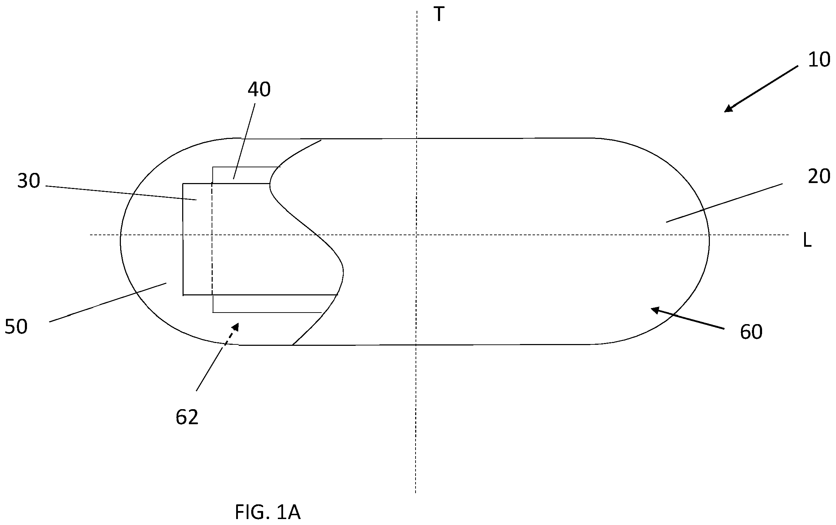

The fluid management layer of the present disclosure may be incorporated into a variety of absorbent articles. An exemplary schematic showing an absorbent article, i.e. a feminine hygiene pad, of the present disclosure is shown in Figure 1A. As shown, absorbent articles 10 in accordance with the present disclosure comprise a topsheet 20, a backsheet 50, and an absorbent core 40 disposed between the topsheet 20 and the backsheet 50. A fluid management layer 30 is disposed between the topsheet 20 and the absorbent core 40. The absorbent article has a wearer-facing surface 60 and an opposing garment-facing surface 62. The wearer-facing surface 60 primarily comprises the topsheet 20 while the garment-facing surface 62 primarily comprises the backsheet 50. Additional components may be included in either the wearer-facing surface 60 and/or the garment-facing surface 62. For example, where the absorbent article is an incontinent pad, a pair of barrier cuffs which extend generally parallel to a longitudinal axis L of the absorbent article 10, may also form a portion of the wearer-facing surface 60. Similarly, a fastening adhesive may be present on the backsheet 50 and form a portion of the garment-facing surface 62 of the absorbent article.

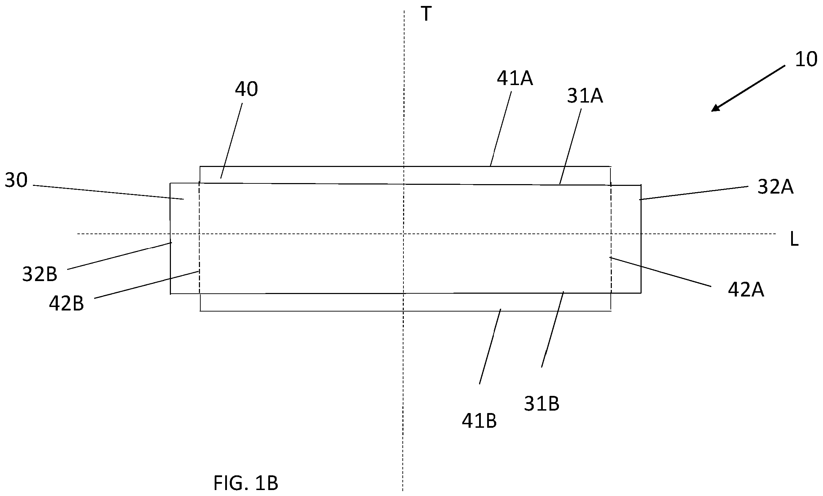

An exemplary configuration for the fluid management layer of the present disclosure is shown in FIG. 1B. As shown, the fluid management layer 30 comprises opposing end edges 32A and 32B which may extend generally parallel to a transverse axis T. And, the fluid management layer 30 comprises side edges 31A and 32B which may extend generally parallel to the longitudinal axis L. Similarly, the absorbent core 40 comprises opposing end edges 42A and 42B which may extend generally parallel to the transverse axis T. And, the absorbent core 40 may comprise side edges 41A and 41B which extend generally parallel to the longitudinal axis L.

As shown, each of the end edges 32A and 32B of the fluid management layer 30 may be disposed longitudinally outboard of the absorbent core 40. However, this is not necessarily required. For example, the end edges 32A and/or 32B may be coextensive with the absorbent core 40 or the end edges 32A and/or 32B may be disposed longitudinally inboard of the end edges 42A and/or 42B of the absorbent core 40.

Similarly, the side edges 31A and/or 31B may be disposed transversely outboard of the side edges 41A and/or 41B of the absorbent core 40. Or, the side edges 31A and/or 31B may be coextensive with the side edges 41A and/or 41B of the absorbent core 40. Additionally, the end edges and/or side edges of the absorbent core and/or fluid management layer may be curvilinear in nature. For example, the side edges of the absorbent core and/or the fluid management layer may curve inward from the ends toward the transverse axis. Such construction may help with conformity of the absorbent article. Similarly, the end edges in conjunction with or independently of the side edges of the absorbent core and/or fluid management layer may comprise a curvilinear path which is either generally concave or generally convex.

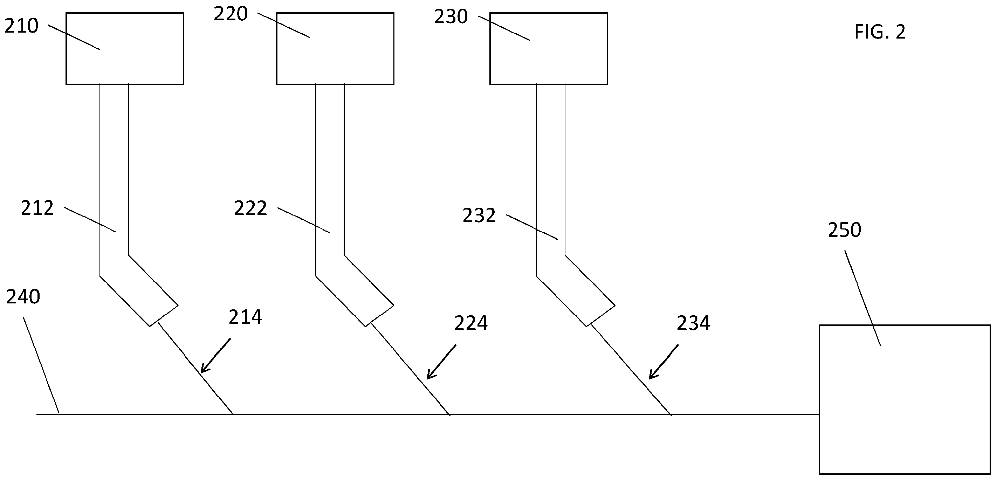

An exemplary process for forming the fluid management layer of the present disclosure is shown in FIG. 2. As shown, a plurality of carding machines 210, 220, and 230 may each create a carded nonwoven web, e.g. 214, 224, and 234, respectively, which is transferred to a carrier belt 240. Each of the carded nonwoven webs 214, 224, and 234, may be provided to the carrier belt 240 via a web chute 212, 222, 232, respectively. It is also worth noting that after the carded nonwoven 214 is deposited on the carrier belt 240, the carded nonwoven 224 is then deposited on the first carded nonwoven 214 on the carrier belt 240. Similarly, the third carded nonwoven web 234 deposited on the second carded nonwoven 224 and the first carded nonwoven 214 on the carrier belt 240. Subsequently, each of the first, second, and third carded nonwoven webs 214, 224, and 234 are then provided to an integration process 250 which utilizes either needles and/or high-pressure water streams to entangle the fibers of the first, second, and third carded nonwoven webs. Both carding and integration processes are well known in the art.

Additional carding machines may be utilized. Additionally, the fluid management layer of the present disclosure may be produced utilizing only two out of the three cards. In such instances, the first carded web 214 would be deposited on the carrier belt 240. And, subsequently, the second carded web 224 would be deposited on the first carded web 214. Then, the first carded web 214 and the second carded web 224 would be integrated as described herein.

It is worth noting that with the arrangement provided in schematic diagram of FIG. 2, a wide variety of configurations for a fluid management layer may be achieved. However, it is important that the fluid management layer of the present disclosure have adequate openness to allow for quick acquisition of fluid yet also are able to lock away liquid insults to reduce the likelihood of rewet. With this in mind, the carded webs, i.e. 214, 224, and/or 234, may be different from one another. For example, one of the carded webs may comprise a different fiber blend than the others. Specifically, assuming the first carded web would be closest to the wearer-facing surface in an absorbent article, the fiber selection for the first carded web 214 may be such that there is more openness associated with this web. The second carded web 224 may be similarly configured. In contrast, the third carded web 234 may be configured collect liquid insults from the void space of the first and second carded webs 214 and 224 and effectively distribute these liquid insults to an absorbent core. Alternatively, the first carded web 214, the second carded web 224 and the third carded web 234 may be configured the same.

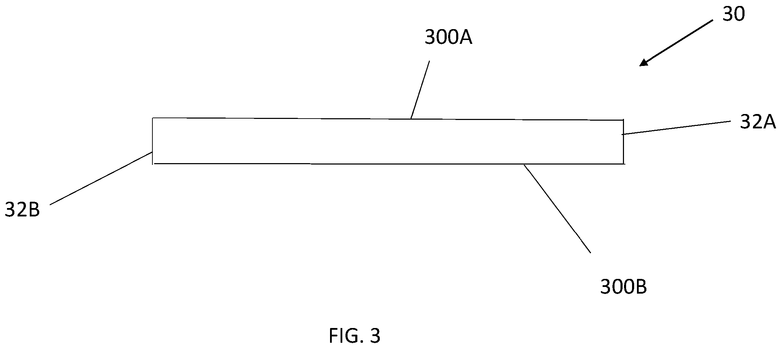

A schematic representation of an exemplary fluid management layer in accordance with the present disclosure is provided in FIG. 3. As shown, the fluid management layer 30 comprises the first surface 300A and the opposing second surface 300B. Between the first surface 300A and the second surface 300B, the fluid distribution layer 30 comprises two or more strata along the Z-direction.

Disposable absorbent articles comprising the fluid management layers of the present disclosure as well as currently marketed fluid management layers were constructed and tested. Specifically, the fluid management layers were removed from their respective articles and tested. For each of the inventive samples and comparative examples, the following was their constituent material makeup.

Comparative example fluid management layer - basis weight of 50 gsm, having 40 percent by weight viscose cellulose fibers having a 1.7 dtex; 20 percent by weight polyethylene terephthalate having a 4.4 dtex; and 40 percent by weight bi-component fibers having a first component of polypropylene and a second component of polyethylene having a 1.7 dtex.

Inventive Sample fluid management layer - basis weight of 55 gsm having 20 percent by weight viscose cellulose fibers having a 1.7 dtex; 30 percent by weight hollow spiral polyethylene terephthalate fibers having a 10 dtex; and 50 percent by weight bi-component fibers having a first component polyethylene terephthalate and polyethylene in a core-sheath configuration where the polyethylene is the sheath.

Table 1 shows caliper of the inventive sample versus the comparative sample. Each of the fluid management layers was removed from their respective finished products.

Table 1

| Sample | Caliper (mm) |
|---|---|
| Comparative Sample | 0.47 |
| Inventive Sample | 0.85 |

As shown, the fluid management layer of the present disclosure is 80 percent thicker than its comparative counterpart with only a 10 percent difference in basis weight. And, as noted previously regarding the caliper factor, for the Inventive Sample is much higher than that of the Comparative Sample as shown in Table 2.

Table 2

| Sample | Caliper Factor (mm/10gsm) |
|---|---|
| Comparative Sample | 0.09 |
| Inventive Sample | 0.15 |

Table 3 shows the acquisition speed of the Inventive Sample versus the Comparative Sample.

Table 3

| Sample | Acquisition Speed (sec) |
|---|---|
| Comparative Sample | 11.16 |
| Inventive Sample | 7.76 |

As shown, the Inventive Sample is 30 percent faster in acquiring liquid insults than the Comparative Sample. The fluid management layers of the present disclosure can exhibit an acquisition speed of less than about 10 seconds, less than about 8 seconds, or most preferably less than about 7 seconds when measured in accordance with the Liquid Strikethrough Time test method described herein. For example, the fluid management layers of the present disclosure can exhibit acquisition times in the range of from between about 5 seconds to about 10 seconds, more preferably from about 5 seconds to about 9 seconds, or most preferably from about 5 seconds to about 8 seconds, specifically reciting all values within these ranges and any ranges created thereby, when measured in accordance with the Liquid Strikethrough Time test method.

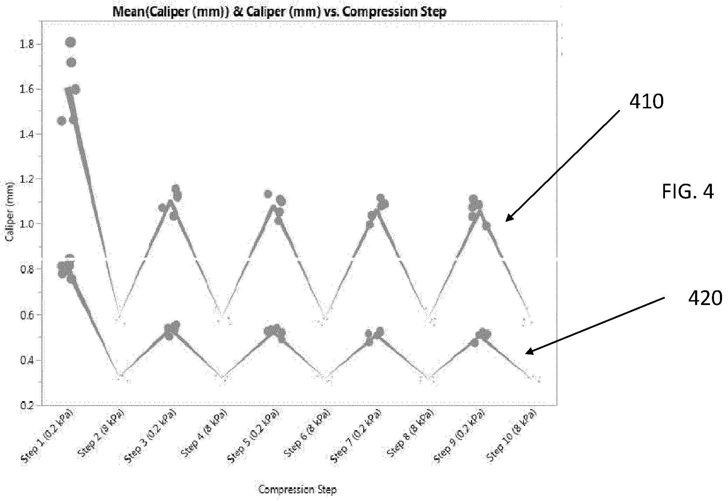

Regarding the soft, cushiony nature of the fluid management layers of the present disclosure, we now refer to FIG. 4. The fluid management layer of the present disclosure is illustrated graphically by the lines associated in group 410 while the comparative sample of the fluid management layer is illustrated graphically by the lines associated in group 420. As shown, the fluid management layer of the present disclosure (Inventive Sample) shows a higher caliper during and after compression versus the Comparative Sample. Even after repeated cycles of compression and relaxation, the Inventive Sample continues to provide a higher caliper. This translates into the Inventive Sample being softer and more cushiony than the Comparative Sample. Data for the graph depicted in FIG. 4 as well as the data in Tables 4A-4C was obtained via the Dynamic Mechanical Analysis method disclosed herein. The data provided in Tables 4A-4C are for the fluid management layers of the Inventive Sample and Comparative Sample and were constructed as noted previously.

Table 4A

| Compression Step | Avg. Inventive Sample Caliper (mm) | Avg. Comparative Sample Caliper (mm) |
|---|---|---|
| Step 1- Caliper (mm) at 0.2 kPa | 1.61 | 0.80 |
| Step 2- Caliper (mm) at 8 kPa | 0.59 | 0.32 |
| Step 3 (Caliper (mm) at 0.2 kPa) | 1.10 | 0.54 |
| Step 4 (8 kPa) | 0.58 | 0.32 |
| Step 5 (Caliper (mm) at 0.2 kPa) | 1.08 | 0.52 |
| Step 6 (8 kPa) | 0.58 | 0.32 |
| Step 7 (Caliper (mm) at 0.2 kPa) | 1.06 | 0.51 |
| Step 8 (8 kPa) | 0.58 | 0.32 |
| Step 9 (Caliper (mm) at 0.2 kPa) | 1.06 | 0.50 |
| Step 10 (8 kPa) | 0.57 | 0.31 |

The data in Table 4A shows that the average caliper of the Inventive Sample is greater than that of the Comparative Sample at every step whether at 0.2 kPa or at 8 kPa. In fact, the data also shows that the average caliper of the Inventive Sample when under compression of 8 kPa is greater than the caliper of the fluid management layer of the Comparative Sample when under a pressure of only 0.2 kPa - except at Step 1.

It is worth noting that the calipers expressed in Table 1 versus those in Table 4A were obtained for different pressures, i.e. 0.5 kPa versus 0.2kPa. As such, the calipers shown are much higher than those listed in Table 1. However, it is also worth noting that from an initial wear standpoint, i.e. at Step 1, the user has a much more cushier absorbent article given the 2X higher caliper of the fluid management layer of the present disclosure, and it is believed that the user can feel the cushiness as shown by the compression in step 2. It is believed that the remainder of the steps, i.e. 3-10, can reflect more of the behavior of the fluid management layer in use - namely the resiliency of the fluid management layers of the present disclosure.

So, for the fluid management layers in accordance with the present disclosure, the caliper at step 1 (measured in accordance with the Dynamic Mechanical Analysis method disclosed herein) can be greater than about 0.9 mm, greater than about 1.2 mm, or most preferably greater than about 1.5 mm. For example, the caliper of the fluid management layer in accordance with the present disclosure can have a caliper (measured in accordance with the Dynamic Mechanical Analysis method disclosed herein) of from between about 1.0 mm to about 2.4 mm, more preferably from about 1.1 mm to about 2.2 mm, or most preferably from about 1.3 mm to about 2.0 mm, specifically reciting all values within these ranges and any ranges created thereby. In order to achieve the 2.4 mm caliper, the basis weight may be between about 60 gsm to about 75 gsm.

For the wearing experience, e.g. steps 2-10, the caliper of the fluid management layer constructed in accordance with the present disclosure can have a caliper (as measured by the Dynamic Mechanical Analysis method disclosed herein) that is greater than about 0.45 mm at 8 kPa and greater than about 0.65 mm at 0.2 kPa, or more preferably greater than about 0.5 mm at 8 kPa and greater than about 0.7 mm at 0.2kPa, or most preferably greater than about 0.60 at 8 kPa and greater than about 0.8 mm at 0.2 kPa. For example, fluid management layers in accordance with the preset disclosure can have a caliper (as measured by the Dynamic Mechanical Analysis method disclosed herein) of from between about 0.45 mm to about 0.9 mm at 8 kPa, more preferably from between about 0.50 mm to about 0.8 mm at 8 kPa, or most preferably from about 0.55 mm to about 0.75 mm at 8 kPa, specifically reciting all values within these ranges and any ranges created thereby. Additionally or independently thereof, the fluid management layers of the present disclosure can have a caliper (as measured by the Dynamic Mechanical Analysis method disclosed herein) of from between about 0.65 mm to about 1.50 mm at 0.2kPa, more preferably from about 0.75 mm to about 1.40 mm at 0.2kPa, or most preferably from about 0.8 mm to about 1.20 mm at 0.2kPa, specifically reciting all values within these ranges and any ranges created thereby.

In Table 4B, the compression distance which is the amount of decrease in caliper between the initial state, e.g. steps 1, 3, 5, 7, and 9, minus the compressed state, i.e. steps 2, 4, 6, 8, and 10.

Table 4B

| Compression Distance (mm) | Avg. Inventive Sample | Avg. Comparative Sample |
|---|---|---|
| Step 1 minus Step 2 (mm) | 1.02 | 0.48 |
| Step 3 minus Step 4 (mm) | 0.52 | 0.22 |
| Step 5 minus Step 6 (mm) | 0.50 | 0.20 |
| Step 7 minus Step 8 (mm) | 0.48 | 0.19 |
| Step 9 minus Step 10 (mm) | 0.49 | 0.19 |
| Average Compression Distance (mm) | 0.50 | 0.20 |

As shown in Table 4B, the fluid management layer of the Inventive Sample also exhibits a greater compression distance than that of the comparative sample. For example, fluid management layers constructed in accordance with the present disclosure may have a compression distance of greater than about 0.60 mm (as measured by the Dynamic Mechanical Analysis method disclosed herein) from Step 1 to step 2. Again, as mentioned above, it is believed that the initial caliper and compression can represent the initial feel that the user gets when using the article. Whereas step 2 onward is believed to be more of a measure for the in-use experience. From step 1 to step 2, the fluid management layer of the present disclosure can exhibit a compression distance of from between about 0.60 mm to about 1.4 mm, more preferably from about 0.7 mm to about 1.4 mm, or most preferably from about 0.8 mm to about 1.4 mm, specifically reciting all values within these ranges and any ranges created thereby - as measured by the Dynamic Mechanical Analysis method disclosed herein.

For the in use steps e.g. 3-10, fluid management layer of the present disclosure can exhibit compression distances of from between about 0.30 mm to about 1.0 mm, more preferably from about 0.40 mm to about 0.8 mm, or most preferably from about 0.45 mm to about 0.7 mm, specifically reciting all values within these ranges and any ranges created thereby - as measured by the Dynamic Mechanical Analysis method disclosed herein.

Table 4C provides additional data regarding the difference in compression distances between the Inventive Sample fluid management layer and the Comparative Sample fluid management layer as well as the percentage difference.

Table 4C

| Compression Distance (mm) | Delta (Inventive - Comparative) | Percent Difference |
|---|---|---|
| Step 1 minus Step 2 (mm) | 0.54 | 212% |
| Step 3 minus Step 4 (mm) | 0.30 | 237% |
| Step 5 minus Step 6 (mm) | 0.29 | 244% |

(continued)

| Compression Distance (mm) | Delta (Inventive - Comparative) | Percent Difference |
|---|---|---|
| Step 7 minus Step 8 (mm) | 0.29 | 249% |
| Step 9 minus Step 10 (mm) | 0.30 | 256% |
| Average Compression Distance (mm) | 0.29 | 246% |

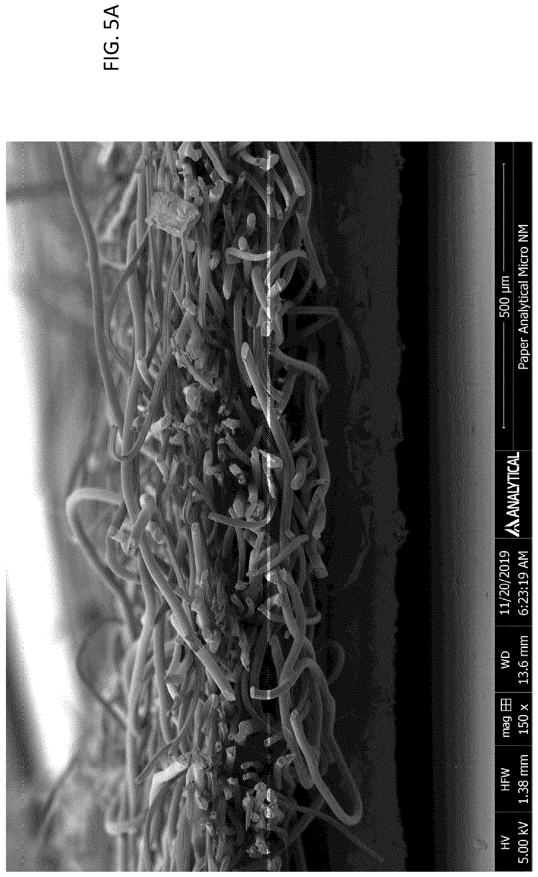

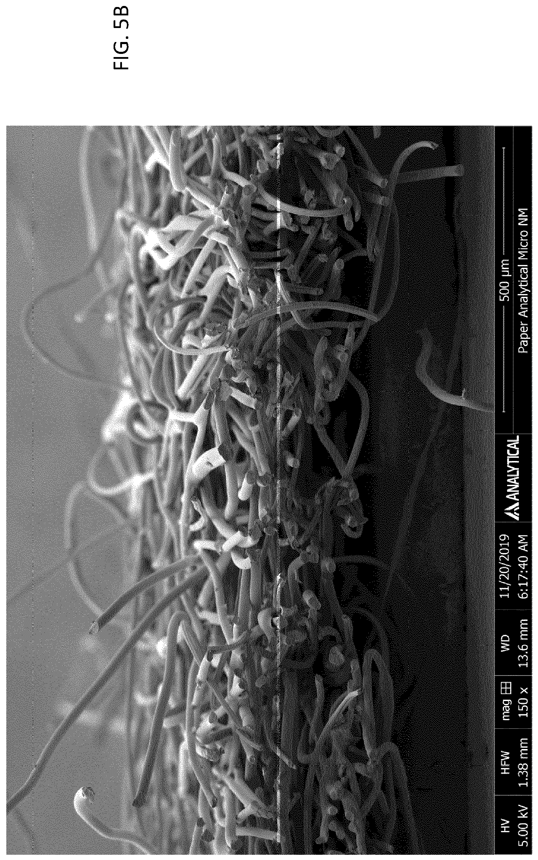

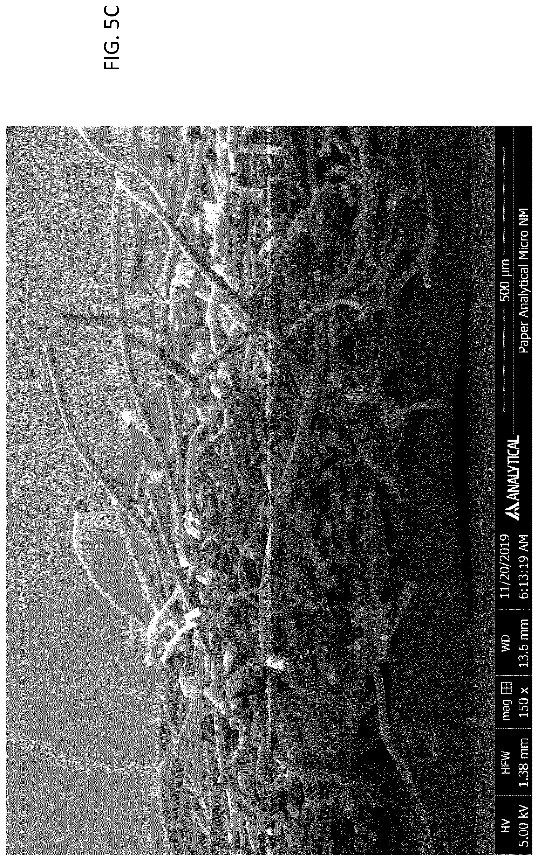

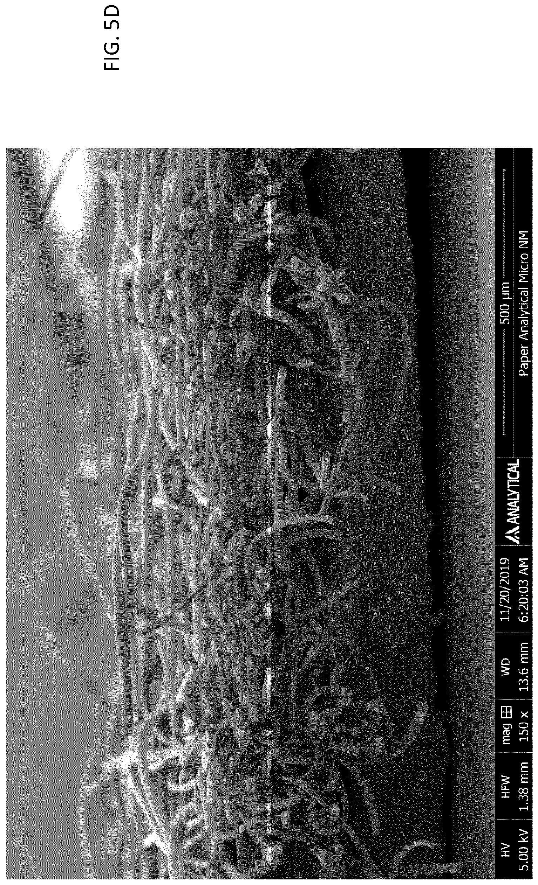

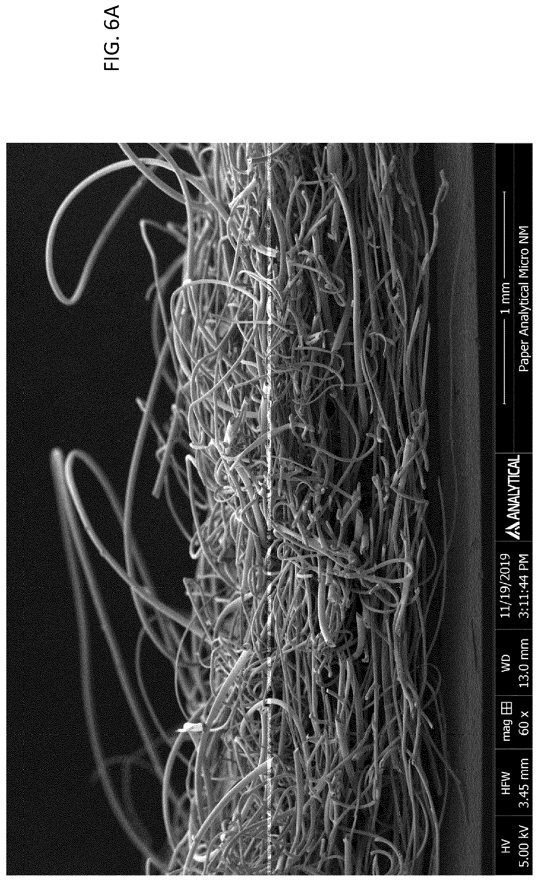

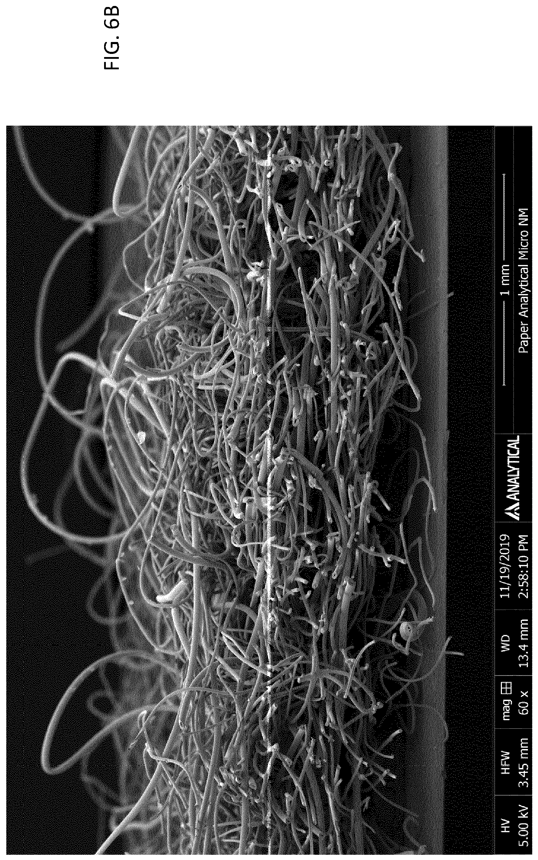

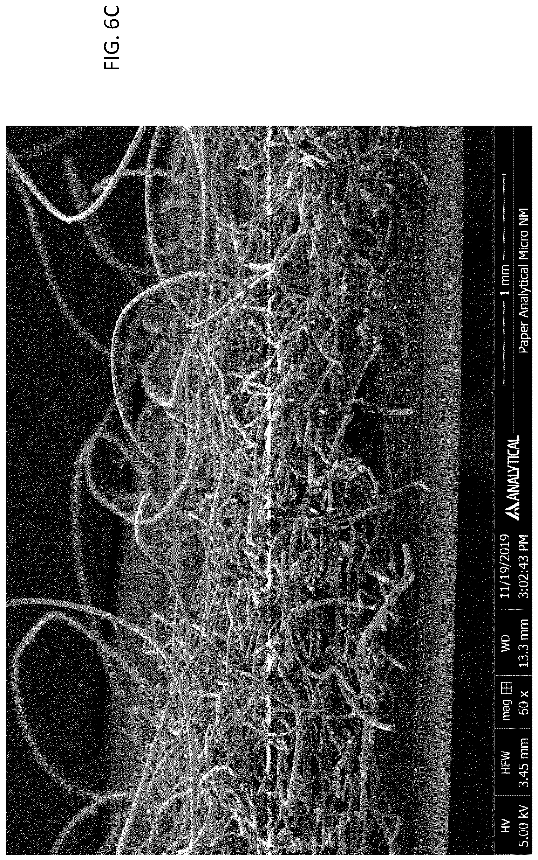

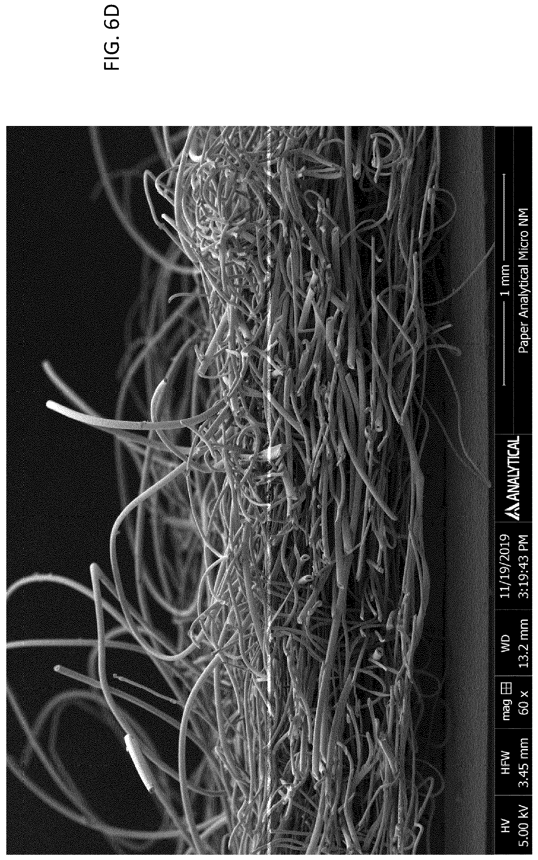

Referring now the FIGS. 5A through 6D, scanning electron microscope images are shown for the Comparative Sample (FIGS. 5A-5D) and for the Inventive Sample, (FIGS. 6A-6D). As shown, viewing the scale indicator on both images, the Inventive Sample has a higher caliper than that of the Comparative Sample. Additionally, it is noted that there are many more fibers associated with the Inventive Sample which extend from a first surface and are disposed, in part, superjacent to the first surface. Moreover, based on the images, many more of the fibers which are disposed, in part, superjacent to the first surface are looped. Namely, these fibers have a first fiber end which extends from the first surface and a second end which extends to the first surface. In contrast, the majority of fibers of the Comparative Sample which are disposed superjacent to the first surface have their respective first ends which extend from the first surface but comprise a second end which is also disposed superjacent to the first surface.

While the fluid management layers of the present disclosure can provide the user with a soft, more cushiony feeling absorbent article, the fluid management layers of the present disclosure also provide the user with the appropriate stiffness such that their resultant absorbent articles can reduce the likelihood of bunching. A metric which can determine the stiffness of a fluid management layer is MD Bending Length. The fluid management layers of the present disclosure may have an MD Bending Length of from between about 4 mN/cm to about 12 mN/cm, specifically reciting all values within these ranges and any ranges created thereby.

Absorbent Articles

Referring back to FIGS. 1A and 1B, as mentioned previously, disposable absorbent articles of the present disclosure may comprise the topsheet 20 and the backsheet 50. Sandwiched therebetween may be the fluid management layer 30 and the absorbent core 40. Additional layers may be positioned between the topsheet 20 and the backsheet 50.

The topsheet 20 may be joined to the backsheet 50 by attachment methods (not shown) such as those well known in the art. The topsheet 20 and the backsheet 50 may be joined directly to each other in the article periphery and may be indirectly joined together by directly joining them to the absorbent core 40, the fluid management layer 30, and/or additional layers disposed between the topsheet 20 and the backsheet 50. This indirect or direct joining may be accomplished by attachment methods which are well known in the art.

The topsheet 20 may be compliant, soft feeling, and non-irritating to the wearer's skin. Suitable topsheet materials include a liquid pervious material that is oriented towards and contacts the body of the wearer permitting bodily discharges to rapidly penetrate through it without allowing fluid to flow back through the topsheet to the skin of the wearer. The topsheet, while being capable of allowing rapid transfer of fluid through it, may also provide for the transfer or migration of the lotion composition onto an external or internal portion of a wearer's skin.

A suitable topsheet 20 can be made of various materials such as woven and nonwoven materials; apertured film materials including apertured formed thermoplastic films, apertured plastic films, and fiber-entangled apertured films; hydro-formed thermoplastic films; porous foams; reticulated foams; reticulated thermoplastic films; thermoplastic scrims; or combinations thereof.

Apertured film materials suitable for use as the topsheet include those apertured plastic films that are non-absorbent and pervious to body exudates and provide for minimal or no flow back of fluids through the topsheet. Nonlimiting examples of other suitable formed films, including apertured and non-apertured formed films, are more fully described in U.S. Patent No. 3,929,135, issued to Thompson on December 30, 1975; U.S. Patent No. 4,324,246, issued to Mullane et al. on April 13, 1982; U.S. Patent No. 4,342,314, issued to Radel et al. on August 3, 1982; U.S. Patent No. 4,463,045, issued to Ahr et al. on July 31, 1984; U.S. Patent No. 5,006,394, issued to Baird on April 9, 1991; U.S. Patent No. 4,609,518, issued to Curro et al. on September 2, 1986; and U.S. Patent No. 4,629,643, issued to Curro et al. on December 16, 1986.

Nonlimiting examples of woven and nonwoven materials suitable for use as the topsheet include fibrous materials made from natural fibers, e.g. cotton, including 100 percent organic cotton, modified natural fibers, synthetic fibers, or combinations thereof. These fibrous materials can be either hydrophilic or hydrophobic, but it is preferable that the topsheet be hydrophobic or rendered hydrophobic. As an option, portions of the topsheet can be rendered hydrophilic, by the use of any known method for making topsheets containing hydrophilic components. Nonwoven fibrous topsheets 20 may be produced by any known procedure for making nonwoven webs, nonlimiting examples of which include spunbonding, carding, wet-laid, air-laid, meltblown, needle-punching, mechanical entangling, thermo-mechanical entangling, and hydroentangling.

The topsheet 20 may be formed from a combination of an apertured film and a nonwoven. For example, a film web and a nonwoven web can be combined as described in U.S. Patent No. 9,700,463. Alternatively, a film may be extruded onto a nonwoven material which is believed to provided enhanced contact between the film layer and the nonwoven material. Exemplary processes for such a combination are described in U.S. Patent Nos. 9,849,602 and 9,700,463.

The backsheet 50 may be positioned adjacent a garment-facing surface of the absorbent core 40 and may be joined thereto by attachment methods such as those well known in the art. For example, the backsheet 50 may be secured to the absorbent core 40 by a uniform continuous layer of adhesive, a patterned layer of adhesive, or an array of separate lines, spirals, or spots of adhesive. Alternatively, the attachment methods may comprise using heat bonds, pressure bonds, ultrasonic bonds, dynamic mechanical bonds, or any other suitable attachment methods or combinations of these attachment methods as are known in the art.

The backsheet 50 may be impervious, or substantially impervious, to liquids (e.g., urine) and may be manufactured from a thin plastic film, although other flexible liquid impervious materials may also be used. As used herein, the term "flexible" refers to materials which are compliant and will readily conform to the general shape and contours of the human body. The backsheet 207 may prevent, or at least inhibit, the exudates absorbed and contained in the absorbent core 205 from wetting articles of clothing which contact the incontinence pad 10 such as undergarments. However, the backsheet 50 may permit vapors to escape from the absorbent core 40 (i.e., is breathable) while in some cases the backsheet 50 may not permit vapors to escape (i.e., non-breathable). Thus, the backsheet 50 may comprise a polymeric film such as thermoplastic films of polyethylene or polypropylene. A suitable material for the backsheet 50 is a thermoplastic film having a thickness of from about 0.012 mm (0.5 mil) to about 0.051 mm (2.0 mils), for example. Any suitable backsheet known in the art may be utilized with the present invention.

The backsheet 50 acts as a barrier to any absorbed bodily fluids that may pass through the absorbent core 40 to the garment surface thereof with a resulting reduction in risk of staining undergarments or other clothing. A preferred material is a soft, smooth, compliant, liquid and vapor pervious material that provides for softness and conformability for comfort, and is low noise producing so that movement does not cause unwanted sound.

Exemplary backsheets are described in US Patent Nos. 5,885,265 (Osborn, III.) issued March 23, 1999; 6,462,251 (Cimini) issued October 8, 2002; 6,623,464 (Bewick-Sonntag) issued September 23, 2003 or US Patent No. 6,664439 (Arndt) issued December 16, 2003. Suitable dual or multi-layer breathable backsheets for use herein include those exemplified in U.S. Pat. No. 3,881,489, U.S. Pat. No. 4,341,216, U.S. Pat. No. 4,713,068, U.S. Pat. No. 4,818,600, EP 203 821, EP 710 471, EP 710 472, and EP 793 952.

Suitable breathable backsheets for use herein include all breathable backsheets known in the art. In principle there are two types of breathable backsheets, single layer breathable backsheets which are breathable and impervious to liquids and backsheets having at least two layers, which in combination provide both breathability and liquid imperviousness. Suitable single layer breathable backsheets for use herein include those described for example in GB A 2184 389, GB A 2184 390, GB A 2184 391, U.S. Pat. No. 4,591,523, U.S. Pat. No. 3 989 867, U.S. Pat. No. 3,156,242 and WO 97/24097.

The backsheet may be a nonwoven web having a basis weight between about 20 gsm and about 50 gsm. In one embodiment, the backsheet is a relatively hydrophobic 23 gsm spunbonded nonwoven web of 4 denier polypropylene fibers available from Fiberweb Neuberger, under the designation F102301001. The backsheet may be coated with a non-soluble, liquid swellable material as described in US Patent No. 6,436,508 (Ciammaichella) issued August 20, 2002.

The backsheet has a garment-facing side and an opposite body-facing side. The garment-facing side of the backsheet comprises a non-adhesive area and an adhesive area. The adhesive area may be provided by any conventional means. Pressure sensitive adhesives have been commonly found to work well for this purpose.

The absorbent core 40 of the present disclosure may comprise any suitable shape including but not limited to an oval, a discorectangle, a rectangle, an asymmetric shape, and an hourglass. For example, in some forms of the present invention, the absorbent core 205 may comprise a contoured shape, e.g. narrower in the intermediate region than in the end regions. As yet another example, the absorbent core may comprise a tapered shape having a wider portion in one end region of the pad which tapers to a narrower end region in the other end region of the pad. The absorbent core 40 may comprise varying stiffness in the MD and CD.

The configuration and construction of the absorbent core 40 may vary (e.g., the absorbent core 40 may have varying caliper zones, a hydrophilic gradient, a superabsorbent gradient, or lower average density and lower average basis weight acquisition zones). Further, the size and absorbent capacity of the absorbent core 40 may also be varied to accommodate a variety of wearers. However, the total absorbent capacity of the absorbent core 40 should be compatible with the design loading and the intended use of the disposable absorbent article or incontinence pad 10.

In some forms of the present invention, the absorbent core 40 may comprise a plurality of multi-functional layers that are in addition to the first and second laminates. For example, the absorbent core 40 may comprise a core wrap (not shown) useful for enveloping the first and second laminates and other optional layers. The core wrap may be formed by two nonwoven materials, substrates, laminates, films, or other materials. In a form, the core wrap may only comprise a single material, substrate, laminate, or other material wrapped at least partially around itself.

The absorbent core 40 of the present disclosure may comprise one or more adhesives, for example, to help immobilize the SAP or other absorbent materials within the first and second laminates.

Absorbent cores comprising relatively high amounts of SAP with various core designs are disclosed in U.S. Pat. No. 5,599,335 to Goldman et al., EP 1,447,066 to Busam et al., WO 95/11652 to Tanzer et al., U.S. Pat. Publ. No. 2008/0312622A1 to Hundorf et al., and WO 2012/052172 to Van Malderen. These may be used to configure the superabsorbent layers.

Additions to the core of the present disclosure are envisioned. In particular, potential additions to the current multi-laminate absorbent core are described in U.S. Pat. No. 4,610,678, entitled "High-Density Absorbent Structures" issued to Weisman et al., on Sep. 9, 1986; U.S. Pat. No. 4,673,402, entitled "Absorbent Articles With Dual-Layered Cores", issued to Weisman et al., on Jun. 16, 1987; U.S. Pat. No. 4,888,231, entitled "Absorbent Core Having A Dusting Layer", issued to Angstadt on Dec. 19, 1989; and U.S. Pat. No. 4,834,735, entitled "High Density Absorbent Members Having Lower Density and Lower Basis Weight Acquisition Zones", issued to Alemany et al., on May 30, 1989. The absorbent core may further comprise additional layers that mimic the dual core system containing an acquisition/distribution core of chemically stiffened fibers positioned over an absorbent storage core as detailed in U.S. Pat. No. 5,234,423, entitled "Absorbent Article With Elastic Waist Feature and Enhanced Absorbency" issued to Alemany et al., on Aug. 10, 1993; and in U.S. Pat. No. 5,147,345. These are useful to the extent they do not negate or conflict with the effects of the below described laminates of the absorbent core of the present invention.

Some examples of a suitable absorbent cores 40 that can be used in the absorbent article of the present disclosure is described in U.S. Patent Application Publication Nos. 2018/0098893 and 2018/0098891.

As noted previously, the absorbent articles comprising the fluid management layers of the present disclosure comprised a storage layer. Referring back to FIGS. 1A and 1B, the storage layer would generally be positioned where the absorbent core 40 is described. The storage layer may be constructed as described regarding absorbent cores. The storage layer can contain conventional absorbent materials. In addition to conventional absorbent materials such as creped cellulose wadding, fluffed cellulose fibers, Rayon fibers, wood pulp fibers also known as airfelt, and textile fibers, the storage layer often includes superabsorbent material that imbibes fluids and form hydrogels. Such materials are also known as absorbent gelling materials (AGM) and may be included in particle form. AGM is typically capable of absorbing large quantities of body fluids and retaining them under moderate pressures. Synthetic fibers including cellulose acetate, polyvinyl fluoride, polyvinylidene chloride, acrylics (such as ORLON), polyvinyl acetate, non-soluble polyvinyl alcohol, polyethylene, polypropylene, polyamides (such as nylon), polyesters, bicomponent fibers, tricomponent fibers, mixtures thereof and the like can also be used in the secondary storage layer. The storage layer can also include filler materials, such as PERLITE, diatomaceous earth, VERMICULITE, or other suitable materials, that lower rewet problems.

The storage layer or fluid storage layer may have absorbent gelling material (agm) in a uniform distribution or may have agm in a non-uniform distribution. The agm may be in the in the form of channels, pockets, stripes, criss-cross patterns, swirls, dots, or any other pattern, either two or three dimensional, that can be imagined by man. The AGM may be sandwiched between a pair of fibrous cover layers. Or AGM may be encapsulated, at least in part, by a single fibrous cover layer.

Portions of the storage layer can be formed only of superabsorbent material or can be formed of superabsorbent materials dispersed in a suitable carrier such as cellulose fibers in the form of fluff or stiffened fibers. One example of a non-limiting storage layer is a first layer formed only of superabsorbent material that is disposed on a second layer that is formed from a dispersion of superabsorbent material within cellulose fibers.

Detailed examples of absorbent cores formed of layers of superabsorbent material and/or layers of superabsorbent material dispersed within cellulose fibers that may be utilized in the absorbent articles (e.g., sanitary napkins, incontinence products) detailed herein are disclosed in U.S. Patent Publication No. 2010/0228209 A1. Absorbent cores comprising relatively high amounts of SAP with various core designs are disclosed in U.S. Pat. No. 5,599,335 to Goldman et al., EP 1,447,066 to Busam et al., WO 95/11652 to Tanzer et al., U.S. Pat. Publ. No. 2008/0312622A1 to Hundorf et al., WO 2012/052172 to Van Malderen, U.S. Pat. No. 8,466,336 to Carlucci, and U.S. Pat. No. 9,693,910 to Carlucci. These may be used to configure the secondary storage layer.

The absorbent article 10 may further comprise barrier cuffs. Some examples of other suitable barrier cuffs are described in U.S. Patent No. 4,695,278; U.S. Patent No. 4,704,115; U.S. Patent No. 4,795,454; U.S. Patent No. 4,909,803; U.S. Patent Application Publication No. 2009/0312730. Additional suitable barrier cuffs are described in U.S. Patent Application Publication Nos. 2018/0098893 and 2018/0098891.

Test Methods

**Caliper**

The caliper, or thickness, of a test specimen is measured as the distance between a reference platform on which the specimen rests and a pressure foot that exerts a specified amount of pressure onto the specimen over a specified amount of time. All measurements are performed in a laboratory maintained at 23 °C $\pm$ 2 C° and 50% $\pm$ 2% relative humidity and test specimens are conditioned in this environment for at least 2 hours prior to testing.

Caliper is measured with a manually-operated micrometer equipped with a pressure foot capable of exerting a steady pressure of 0.50 kPa $\pm$ 0.01 kPa onto the test specimen. The manually-operated micrometer is a dead-weight type instrument with readings accurate to 0.01 mm. A suitable instrument is Mitutoyo Series 543 ID-C Digimatic, available from VWR International, or equivalent. The pressure foot is a flat ground circular movable face with a diameter that is smaller than the test specimen and capable of exerting the required pressure. A suitable pressure foot has a diameter of 25.4 mm, however a smaller or larger foot can be used depending on the size of the specimen being measured. The test specimen is supported by a horizontal flat reference platform that is larger than and parallel to the surface of the pressure foot. The system is calibrated and operated per the manufacturer's instructions.

Obtain a test specimen by removing it from an absorbent article, if necessary. When excising the test specimen from an absorbent article, use care to not impart any contamination or distortion to the test specimen layer during the process. The test specimen is obtained from an area free of folds or wrinkles, and it must be larger than the pressure foot.

To measure caliper, first zero the micrometer against the horizontal flat reference platform. Place the test specimen on the platform with the test location centered below the pressure foot. Gently lower the pressure foot with a descent rate of 3.0 mm $\pm$ 1.0 mm per second until the full pressure is exerted onto the test specimen. Wait 5 seconds and then record the caliper of the test specimen to the nearest 0.001 mm. In like fashion, repeat for a total of ten replicate test specimens. Calculate the arithmetic mean for all caliper measurements and report as Caliper to the nearest 0.001 mm.

**Caliper factor**

The caliper factor, as mentioned previously is the caliper per 10 gsm of basis weight of the sample. So, the equation is caliper / (basis weight/10).

**Basis Weight**

The basis weight of a test sample is the mass (in grams) per unit area (in square meters) of a single layer of material and is measured in accordance with compendial method WSP 130.1. The mass of the test sample is cut to a known area, and the mass of the sample is determined using an analytical balance accurate to 0.0001 grams. All measurements are performed in a laboratory maintained at 23 °C $\pm$ 2 C° and 50% $\pm$ 2% relative humidity and test samples are conditioned in this environment for at least 2 hours prior to testing.

Measurements are made on test samples taken from rolls or sheets of the raw material, or test samples obtained from a material layer removed from an absorbent article. When excising the material layer from an absorbent article, use care to not impart any contamination or distortion to the layer during the process. The excised layer should be free from residual adhesive. To ensure that all adhesive is removed, soak the layer in a suitable solvent that will dissolve the adhesive without adversely affecting the material itself. One such solvent is THF (tetrahydrofuran, CAS 109-99-9, for general use, available from any convenient source). After the solvent soak, the material layer is allowed to thoroughly air dry in such a way that prevents undue stretching or other deformation of the material. After the material has dried, a test specimen is obtained. The test specimen must be as large as possible so that any inherent material variability is accounted for.

Measure the dimensions of the single layer test specimen using a calibrated steel metal ruler traceable to NIST, or equivalent. Calculate the Area of the test specimen and record to the nearest 0.0001 square meter. Use an analytical balance to obtain the Mass of the test specimen and record to the nearest 0.0001 gram. Calculate Basis Weight by dividing Mass (in grams) by Area (in square meters) and record to the nearest 0.01 grams per square meter (gsm). In like fashion, repeat for a total of ten replicate test specimens. Calculate the arithmetic mean for Basis Weight and report to the nearest 0.01 grams/square meter.

**Material Compositional Analysis**

The quantitative chemical composition of a test specimen comprising a mixture of fiber types is determined using ISO 1833-1. All measurements are performed in a laboratory maintained at 23 °C $\pm$ 2 C° and 50% $\pm$ 2% relative humidity.

Analysis is performed on test samples taken from rolls or sheets of the raw material, or test samples obtained from a material layer removed from an absorbent article. When excising the material layer from an absorbent article, use care to not impart any contamination or distortion to the layer during the process. The excised layer should be free from residual adhesive. To ensure that all adhesive is removed, soak the layer in a suitable solvent that will dissolve the adhesive without adversely affecting the material itself. One such solvent is THF (tetrahydrofuran, CAS 109-99-9, for general use, available from any convenient source). After the solvent soak, the material layer is allowed to thoroughly air dry in such a way that prevents undue stretching or other deformation of the material. After the material has dried, a test specimen is obtained and tested as per ISO 1833-1 to quantitatively determine its chemical composition.

Fiber Decitex (Dtex)

Textile webs (e.g. woven, nonwoven, airlaid) are comprised of individual fibers of material. Fibers are measured in terms of linear mass density reported in units of decitex. The decitex value is the mass in grams of a fiber present in 10,000 meters of that fiber. The decitex value of the fibers within a web of material is often reported by manufacturers as part of a specification. If the decitex value of the fiber is not known, it can be calculated by measuring the cross-sectional area of the fiber via a suitable microscopy technique such as scanning electron microscopy (SEM), determining the composition of the fiber with suitable techniques such as FT-IR (Fourier Transform Infrared) spectroscopy and/or DSC (Dynamic Scanning Calorimetry), and then using a literature value for density of the composition to calculate the mass in grams of the fiber present in 10,000 meters of the fiber. All testing is performed in a room maintained at a temperature of 23° C $\pm$ 2.0° C and a relative humidity of 50% $\pm$ 2% and samples are conditioned under the same environmental conditions for at least 2 hours prior to testing.

If necessary, a representative sample of web material of interest can be excised from an absorbent article. In this case, the web material is removed so as not to stretch, distort, or contaminate the sample.

SEM images are obtained and analyzed as follows to determine the cross-sectional area of a fiber. To analyze the cross section of a sample of web material, a test specimen is prepared as follows. Cut a specimen from the web that is about 1.5 cm (height) by 2.5 cm (length) and free from folds or wrinkles. Submerge the specimen in liquid nitrogen and fracture an edge along the specimen's length with a razor blade (VWR Single Edge Industrial Razor blade No. 9, surgical carbon steel). Sputter coat the specimen with gold and then adhere it to an SEM mount using double-sided conductive tape (Cu, 3M available from electron microscopy sciences). The specimen is oriented such that the cross section is as perpendicular as possible to the detector to minimize any oblique distortion in the measured cross sections. An SEM image is obtained at a resolution sufficient to clearly elucidate the cross sections of the fibers present in the specimen. Fiber cross sections may vary in shape, and some fibers may consist of a plurality of individual filaments. Regardless, the area of each of the fiber cross sections is determined (for example, using diameters for round fibers, major and minor axes for elliptical fibers, and image analysis for more complicated shapes). If fiber cross sections indicate inhomogeneous cross-sectional composition, the area of each recognizable component is recorded and dtex contributions are calculated for each component and subsequently summed. For example, if the fiber is bi-component, the cross-sectional area is measured separately for the core and sheath, and dtex contribution from core and sheath are each calculated and summed. If the fiber is hollow, the cross-sectional area excludes the inner portion of the fiber comprised of air, which does not appreciably contribute to fiber dtex. Altogether, at least 100 such measurements of cross-sectional area are made for each fiber type present in the specimen, and the arithmetic mean of the cross-sectional area $a_k$ for each are recorded in units of micrometers squared ($\mu m^2$) to the nearest 0.1 $\mu m^2$.

Fiber composition is determined using common characterization techniques such as FTIR spectroscopy. For more complicated fiber compositions (such as polypropylene core/polyethylene sheath bi-component fibers), a combination of common techniques (e.g. FTIR spectroscopy and DSC) may be required to fully characterize the fiber composition. Repeat this process for each fiber type present in the web material.

The decitex $d_k$ value for each fiber type in the web material is calculated as follows:

$$d_k = 10\,000\,\text{m} \times a_k \times \rho_k \times 10^{-6}$$

where $d_k$ is in units of grams (per calculated 10,000 meter length), $a_k$ is in units of $\mu m^2$, and $\rho_k$ is in units of grams per cubic centimeter (g/cm$^3$). Decitex is reported to the nearest 0.1 g (per calculated 10,000 meter length) along with the fiber type (e.g. PP, PET, cellulose, PP/PET bico).

**Dynamic Mechanical Analysis**

A Dynamic Mechanical Analyzer (DMA) is used to measure compression resistance and recovery properties of test specimens obtained from an individual material or a portion of an absorbent article. A suitable instrument is the DMA Q800 (available from TA Instruments, New Castle, Delaware), or equivalent, equipped with 40 mm diameter compression plates. The test specimen is exposed to a series of axial compression force ramps with controlled changes in stress, and the resultant change in displacement is measured. All testing is performed in a room controlled at 23°C $\pm$ 3C° and 50% $\pm$ 2% relative humidity.

Samples are conditioned at 23°C $\pm$ 3C° and 50% $\pm$ 2% relative humidity for at least 2 hours before testing. When testing a whole article, remove the release paper from any panty fastening adhesive on the garment facing side of the article if present. Lightly apply talc powder to the adhesive to mitigate any tackiness. Place the article, body facing surface up, on a bench, then identify and mark the test location as follows. For symmetrical articles (i.e. the front of the article is the same shape and size as the back of the article when laterally divided along the midpoint of the longitudinal axis of the sample), the test location is the intersection of the midpoints of the longitudinal axis and lateral axis of the article. For asymmetrical articles (i.e. the front of the article is not the same shape and size as the back of the article when laterally divided along the midpoint of the longitudinal axis of the article), the test location is the intersection of the midpoint of the longitudinal axis of the article and a lateral axis positioned at the midpoint of the article's wings. Using a round cutting die, cut a 40 mm diameter test specimen centered at the test location. When testing an individual material layer (e.g. a raw material or a layer excised from an article), the test location is determined in the same manner as when testing a whole article based upon where the individual material would be located within said article.

Program the DMA for a controlled force test with force ramps from 0.02 N to 10 N at a rate of 25 N/minute. The test temperature is ambient (23°C $\pm$ 3C°), therefore the furnace is left open. Set the Poisson's ratio to 0.44. The data sampling interval is 0.1 s/pt. Data is collected over 5 cyclic force ramps, e.g. 5 force ramps from 0.2 N to 10 N and 5 force ramps from 10.00 N to 0.02 N. The initial thickness of the test specimen is recorded by the instrument when the initial force applied is 0.02 N.

Start the test and collect Force (N) and displacement (mm) data for all five cyclic force ramps. The first half the cycle is the compression step (where force is being applied to the test specimen) whereas the second half of the cycle is the recovery step (where force is being removed from the test specimen). The following intermediate results are calculated for the test specimen.

| | |
|---|---|
| Displacement [mm] | Total displacement for each row<br>(abs(actual specimen thickness - current specimen thickness)) |
| Engineering Strain [] | displacement / initial length |
| Engineering Stress [Pa] | static force / specimen area<br>(specimen area: $\pi*(0.04*0.5)^2(\sim0.001257m^2)$) |
| Work Increment [mJ] | Approximation using trapezoid rule - change in thickness times mean force<br>((x2 - x1) * ½ *(y1 + y2)) |
| Work (by Step#) [mJ] | Approximated area under the force-displacement curve for each step<br>(sum of Work Increments, per step) |
| Energy dissipation (by Cycle#) [mJ] | Approximated hysteresis area of the force-displacement curve<br>(sum of Work Increments, per cycle) |
| min Thickness (by step#) | Minimum specimen thickness per step |
| max Thickness (by step#) | Maximum specimen thickness per step |
| Recovery | recovery based on initial length (cycle 1) |

Now for each individual force ramp Cycle (1 - 5), the following parameters are calculated and reported.

| | |
|---|---|
| initial length* [mm] | Specimen thickness* at the begin of each cycle |
| length after compression [mm] | Specimen thickness at the maximum force within each cycle |
| length after recovery [mm] | Specimen thickness at the end of each cycle |
| max compression [] | Compression strain at maximum force<br>((initial length - length after recovery) / initial length) |
| recovery [] | Recovery within a cycle<br>(1 - ((length after recovery - initial length) / (length after compression - initial length))) |
| modulus at 8kPa [MPa] | Secant modulus<br>(maximum stress = maximum force / specimen area; Modulus = maximum stress / max compression) |
| compression work [mJ] | Approximated area under the force-displacement curve of the Compression step of the cycle |

(continued)

| | (sum of incremental area in the compression portion of the cycle using trapezoid rule (x2 - x1) * ½ *(y1 + y2)) |
|---|---|
| recovery work [mJ] | Approximated area under the force-displacement curve of the Recovery step of the cycle<br><br>(sum of incremental area in the recovery portion of the cycle using trapezoid rule (x2 - x1) * ½ *(y1 + y2)) |
| Energy Dissipation [mJ] | Approximated hysteresis area of the force-displacement curve<br><br>(sum of incremental area for the whole cycle using trapezoid rule (x2 - x1) * ½ *(y1 + y2)) |
| Compliance [1/Pa] | Inverse of secant modulus<br><br>(1 / max compression) |

In like fashion, repeat for a total of five replicate test specimens and report the arithmetic mean for each calculated parameter.

**Liquid Strikethrough Time**

Strikethrough time is measured for a test sample insulted with a known volume of test liquid, using a strikethrough plate and an electronic circuit interval timer according to compendial method WSP 70.3. The time required for the test liquid to pass through the test sample is recorded. All measurements are performed in a laboratory maintained at 23 °C ± 2 C° and 50% ± 2% relative humidity and test samples are conditioned in this environment for at least 2 hours prior to testing.

The materials required to execute this test are as follows. The test liquid is 0.9% saline (prepared by weighing 9.0 g ± 0.05g of reagent grade NaCl into a weigh boat, transferring it into a 1L volumetric flask and diluting to volume with de-ionized water). The Standard Absorbent Pad that is placed beneath the test sample consists of 5 layers of Ahlstrom Grade 989 filter paper (available from Ahlstrom-Munksj o North America LLC, Alpharetta, GA), or equivalent, cut to 10 cm by 10 cm. The strikethrough plate and electronic circuit interval timer are described within the WSP method, and can be purchased from W. Fritz Mezger, Inc (Spartanburg, SC) as the Lister AC Strikethrough Tester.

Measurements are made on test samples taken from rolls or sheets of raw material, cut to a size of 10 cm by 10 cm. Measurements can also be made on test samples obtained from a material layer removed from an absorbent article. When excising the material layer from an absorbent article, use care to not impart any contamination or distortion to the layer during the process. If the material layer has been excised from an absorbent article, a test location must be determined and marked as follows. For symmetrical articles (i.e. the front of the article is the same shape and size as the back of the article when laterally divided along the midpoint of the longitudinal axis of the article), the test location is the intersection of the midpoints of the longitudinal axis and lateral axis of the article. For asymmetrical samples (i.e. the front of the article is not the same shape and size as the back of the article when laterally divided along the midpoint of the longitudinal axis of the article), the test location is the intersection of the midpoint of the longitudinal axis of the article and a lateral axis positioned at the midpoint of the article's wings. The entire layer excised from an absorbent article is the test sample in this case, and it is not cut down to a specific size. In this case, it is possible that the width of the test sample may be smaller than 10 cm, however it must be wide enough at the test location to entirely cover the opening of the strikethrough plate.

The test sample is placed onto the filter paper with the side intended to face the body of the wearer facing up and the test location centered at the midpoint of the filter paper. The strikethrough plate is then centered over the test sample and filter paper, and the test is executed as per WSP 70.3. Only a single gush of test liquid is applied, and the strikethrough time is recorded to the nearest 0.01 seconds.

In like fashion, the test is repeated for five replicate test samples, using a fresh stack of filter paper for each replicate. Calculate and report the Strikethrough Time as the arithmetic mean of the replicates to the nearest 0.01 sec.

**Wet CD Flexibility - Material Layer**

Wet CD flexibility is a measure of the force required to deform an individual material layer taken from absorbent article loaded with a known volume of Paper Industry Fluid (PIF), as described herein, using a cyclic compression test on a horizontally oriented constant rate of extension tensile tester with a computer interface. The test consists of 7 cycles of load application and load removal, and the average hysteresis area (Flexibility), average initial slope (Initial Stiffness) and average total slope (Total Stiffness) of the force vs displacement curves over the last 3 cycles is calculated. All testing is performed in a room controlled at 23°C ± 3C° and 50% ± 2% relative humidity.

The tensile tester is equipped with a load frame that consists of two guide profiles, two lead screws, and two moving crossheads (each mounted directly opposite the other). The crossheads are driven symmetrically in opposing directions by two lead screws with a play-free precision ball screw guided by a linear guide via two carriages on ball bearings. A suitable instrument can be purchased from Zwick Roell (Ulm, Germany) as item D0724788. The instrument is calibrated and operated according to the manufacturer's instructions. A single load cell, for which forces measured are within 10% to 90% of the limit of the cell, is mounted on one of the moving crossheads. The tensile tester is equipped with an identical set of rounded-edge grips that are used to hold the test specimen, with one attached to the moving crosshead on the right and the other attached to the moving crosshead on the left, both centrally aligned with the pull axis of the tensile tester. The rounded-edge grips have a hemisphere-shape with a radius of 50 mm and are constructed in such a way that the test specimen can be gripped securely to prevent slippage. Such grips are available from Zwick Roell (Ulm, Germany). The right and left grips are mounted in such a way that they are horizontally and vertically aligned.

Program the tensile tester for a compression test, collecting force (N) and displacement (mm) data at an acquisition rate of 50 Hz as the crosshead travels at a rate of 150 mm/min. The gauge length is set to 55 mm (separation between the outermost edges of the hemispherical grips), with a path length of 20 mm. The compression test consists of seven force cycles. In the first cycle, the grips move from a starting separation distance of 55 mm to a separation distance of 35 mm, and then return to a separation distance of 50 mm. For each of the subsequent 6 cycles, the grips move from a separation distance of 50 mm to 35 mm (force application) and then back to a separation distance of 50 mm (force removal).

The test articles are conditioned at 23 °C ± 2 C° and 50% ± 2% relative humidity for at least 2 hours prior to testing. After conditioning, remove the article from any wrapper present, but leave the PFA (Panty Fastening Adhesive) protective cover in place. If the article is folded, gently unfold it and smooth out any wrinkles. If wings are present, unfold them but leave the release paper intact. Place the article on a horizontally flat rigid surface with the body side facing up and the wings extended. Now determine and mark the dosing location as follows. Mark an area that is 40 mm long (aligned with the longitudinal axis of article) by 30 mm wide (aligned with the lateral axis of the article) that is centered about the intersection of the midpoints of the longitudinal axis and lateral axis of the article.

Dose the article with the test liquid as follows. The article is loaded with PIF using a mechanical pipette. Accurately and evenly distribute 7.5 mL of PIF throughout the pre-marked dose location within 5 seconds without splashing. As soon as the PIF has been dispensed from the pipette, start a 10 minute timer. Ensure the tensile tester is programmed and the grips are spaced 55 mm apart, as previously described. After 10 minutes have expired, prepare the test specimen as follows. Carefully excise the entire material layer to be tested from the article in such a way that no distortion or compression is imparted to the layer and the liquid within the layer is undisturbed. Note the dose location on the test specimen layer that is excised. Now insert the lateral sides of the test specimen into the grips of the tensile tester, ensuring that the specimen is centrally aligned at the dose location in both the longitudinal and lateral directions. The lateral axis of the test specimen at the longitudinal midpoint is exactly aligned with the central pull axis of the tensile tester. Zero the load cell and start the cyclic compression test, collecting force (N) and displacement (mm) data for all 7 cycles of force application and force removal.

Construct a graph of force (N) versus displacement (mm) for the last 3 cycles (cycles 5 - 7). Calculate the area of the hysteresis loop for each of the 3 cycles (the entire area created during the load application and load removal) and record to the nearest 0.01 N*mm. Now calculate the average area across all 3 cycles and record as Wet CD Flexibility to the nearest 0.01 N*mm. For each of the 3 cycles, determine the initial slope of the line between displacement values of 5.25 mm and 5.50 mm (during the load application portion of the cycle) and record to the nearest 0.1 N/m. Now calculate the average initial slope across all 3 cycles and record as Initial Stiffness to the nearest 0.1 N/mm. For each of the 3 cycles, determine the total slope of the line between the point that occurs at the minimum force (from the load application portion of the cycle) and the point that occurs at the maximum force (from the load removal portion of the cycle), and record to the nearest 0.1 N/m. Now calculate the average total slope across all 3 cycles and report as Total Stiffness to the nearest 0.1 N/m.

In like fashion, repeat the test for a total of ten replicate test samples. Calculate the arithmetic mean for Wet CD Flexibility and report to the nearest 0.01 N*mm. Calculate the arithmetic mean for Wet CD Initial Stiffness and report to the nearest 0.1 N/m. Calculate the arithmetic mean for Wet CD Total Stiffness and report to the nearest 0.1 N/m.

**Paper Industry Fluid (PIF) Preparation**

Paper Industry Fluid (PIF) is a widely accepted non-hazardous, non-blood based surrogate fluid for human menses. PIF is an aqueous mixture composed of sodium chloride, carboxymethyl cellulose, glycerol and sodium bicarbonate, and the surface tension is adjusted with the addition of a nonionic surfactant. This standard test fluid was developed by the technical committee of the French industry group of producers of sanitary products (*Groupment Francaise de producteurs d'articles pour usage sanitaires et domestiques*) and described in the AFNOR standard *Normilization francaise* Q34-018 of September 1994. When properly prepared, PIF has a viscosity of 11 ± 1 centipoise, a surface tension of 50 ± 2 mN/m and a pH value of 8 ± 1 at a temperature of 23 °C ± 1 C°.

Viscosity of the prepared PIF is performed using a low viscosity rotary viscometer (a suitable instrument is the Cannon LV-2020 Rotary Viscometer with UL adapter, Cannon Instrument Co., State College, PA, or equivalent). The appropriate size spindle for the viscosity range is selected, and the instrument is operated and calibrated as per the manufacturer's instructions. Measurements are taken at 23 °C ± 1 C° and at 30 rpm. Results are reported to the nearest 0.1 centipoise.

Surface tension of the prepared PIF is performed using a tensiometer. A suitable instrument is the Kruss K100 with a plate method (available from Kruss GmbH, Hamburg, Germany), or equivalent. The instrument is operated and calibrated as per the manufacturer's instructions. Measurements are taken when the aqueous mixture is at a temperature of 23 °C ± 1 C°. Results are reported to the nearest 0.1 mN/m.

Reagents needed for the PIF preparation include: sodium chloride (reagent grade solid), carboxymethyl cellulose (> 98% purity, mass fraction), glycerol (reagent grade liquid), sodium bicarbonate (reagent grade solid), a 0.25% wt aqueous solution of polyethylene glycol *tert*-octylphenyl ether (Triton™ X-100; reagent grade) and deionized water, each reagent available from VWR International or equivalent source.

The following preparation steps will result in about 1 liter of PIF. Add 80.0 ± 0.01 g of glycerol to a 2 L glass beaker. The amount of carboxymethyl cellulose (CMC) directly affects the final viscosity of the prepared PIF, so the amount of CMC is adjusted to yield a final viscosity within the target range (11 ± 1 centipoise). Carboxymethyl cellulose is slowly added to the beaker of glycerol (in an amount between 15 - 20 grams) while stirring to minimize clumping. Continue stirring for about 30 minutes or until all of the CMC has dissolved and no clumps remain. Now add 1000 + 1 g of deionized water to the beaker and continue to stir. Next add 10.0 + 0.01 g of sodium chloride and 4.0 + 0.01 g of sodium bicarbonate to the beaker while stirring. The amount of nonionic surfactant solution (0.25% wt Triton™ X-100 aqueous) directly affects the final surface tension of the prepared PIF, so the amount of 0.25% wt Triton™ X-100 is adjusted to yield a final surface tension within the target range (50 ± 2 mN/m). The total amount of 0.25% wt Triton X-100 solution to add to the beaker is around 3.7 mL.

Ensure the temperature of the prepared PIF is at 23 °C ± 1 C°. Using the viscosity and surface tension methods previously described, ensure the viscosity is 11 ± 1 centipoise and the surface tension is 50 ± 2 mN/m. Measure the pH of the prepared PIF using pH strips or a pH meter (any convenient source) and ensure the pH is within the target range (8 ± 1). If the prepared batch of PIF does not meet the specified targets, it is disposed and another batch is made adjusting the amounts of CMC and 0.25% wt Triton™ X-100 solution as appropriate.

The qualified batch of PIF is stored covered at 23 °C ± 1 C°. The viscosity, surface tension and pH are tested daily prior to use to ensure the mixture meets the specified targets for each parameter.

**MD Bending Length**

The measurements for MD Bending Length provided herein were obtained by using Worldwide Strategic Partners (WSP) Test Method 90.1.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. A fluid management layer comprising an integrated, carded nonwoven having a basis weight of between about 40 grams per square meter (gsm) and about 75 gsm as determined by the Basis Weight method detailed herein, and comprising a plurality of absorbent fibers, a plurality of stiffening fibers and a plurality of resilient fibers, wherein the fluid management layer has a caliper factor of at least about 0.13 as determined by the Caliper and Caliper Factor methods detailed herein;
   wherein the absorbent fibers are present in an amount of between about 10 percent by weight to about 60 percent by weight, the resilient fibers are present in an amount of between about 15 percent by weight to about 70 percent by weight, and the stiffening fibers are present in an amount of between about 25 percent by weight to about 70 percent by weight as determined by the Material Composition Analysis method detailed herein; wherein the fluid management layer is a nonwoven integrated by needlepunching.

2. The fluid management layer of claim 1, wherein the nonwoven exhibits a fluid acquisition time of less than 10 second as measured by the Liquid Strikethrough Time method detailed herein.

3. The fluid management layer of any of the preceding claims, wherein the nonwoven has a caliper factor from between about 0.13 mm to about 0.3 mm or more preferably from about 0.15 mm to about 0.25 mm as determined by the Caliper and Caliper Factor methods detailed herein.

4.  The fluid management layer of any of the preceding claims, having a basis weight of between about 45 gsm to about 70 gsmas determined by the Basis Weight method detailed herein.

5.  The fluid management layer of any of the preceding claims, wherein the weight percentage of absorbent fibers is between 20 percent to about 30 percent.

6.  The fluid management layer of any of the preceding claims, wherein the resilient fibers comprise a linear density of from about 4 dtex to about 15 dtex, more preferably from about 5 dtex to about 12 dtex, or most preferably from about 6 dtex to about 10 dtex as determined by the Fiber Decitex method detailed herein.

7.  The fluid management layer of any of the preceding claims, wherein the stiffening fibers comprise a linear density of from between about 1.0 dtex to about 6 dtex, more preferably from about 1.5 dtex to about 5 dtex, or most preferably from about 2.0 dtex to about 4 dtex as determined by the Fiber Decitex method detailed herein.

8.  The fluid management layer of any of claims 1-5, wherein the weight percentage of the absorbent fibers is between 30 percent to about 60 percent.

9.  The fluid management layer of claim 8, wherein the resilient fibers comprise a linear density of from about 5 dtex to about 15 dtex, more preferably from about 6 dtex to about 12 dtex, or most preferably from about 7 dtex to about 10 dtex as determined by the Fiber Decitex method detailed herein.

10.  The fluid management layer of claims 8 and 9, wherein the stiffening fibers comprise a linear density of from about 3 dtex to about 6 dtex, more preferably from about 4 dtex to about 6 dtex as determined by the Fiber Decitex method detailed herein.

11.  A disposable absorbent article comprising a topsheet, a backsheet, an absorbent core disposed between the topsheet and the backsheet, the disposable absorbent article further comprising the fluid management layer of any of the preceding claims.

12.  The disposable absorbent article of claim 11, wherein the fluid management layer is disposed between the topsheet and the absorbent core.

**Patentansprüche**

1.  Fluidmanagementschicht, umfassend einen integrierten, kardierten Vliesstoff, die ein Flächengewicht von zwischen etwa 40 Gramm pro Quadratmeter (g/m$^2$) und etwa 75 g/m$^2$ aufweist, wie durch das hierin eingehend beschriebene Flächengewichtsverfahren bestimmt, und umfassend eine Vielzahl von Absorptionsfasern, eine Vielzahl von Versteifungsfasern und eine Vielzahl von elastischen Fasern, wobei die Fluidmanagementschicht einen Stärkefaktor von wenigstens etwa 0,13 aufweist, wie durch die hierin eingehend beschriebenen Stärke- und Stärkefaktorverfahren bestimmt;

    wobei die Absorptionsfasern in einer Menge von zwischen etwa 10 Gewichtsprozent bis etwa 60 Gewichtsprozent gegenwärtig sind, die elastischen Fasern in einer Menge von zwischen etwa 15 Gewichtsprozent bis etwa 70 Gewichtsprozent gegenwärtig sind und die Versteifungsfasern in einer Menge von zwischen etwa 25 Gewichtsprozent bis etwa 70 Gewichtsprozent gegenwärtig sind, wie durch das hierin eingehend beschriebene Materialzusammensetzungsanalyseverfahren bestimmt;
    wobei die Fluidmanagementschicht ein Vliesstoff ist, der durch ein Vernadeln integriert wird.

2.  Fluidmanagementschicht nach Anspruch 1, wobei der Vliesstoff eine Fluidaufnahmezeit von weniger als 10 Sekunden vorweist, wie durch das hierin eingehend beschriebene Flüssigkeitsdurchdringungszeitverfahren gemessen.

3.  Fluidmanagementschicht nach einem der vorstehenden Ansprüche, wobei der Vliesstoff einen Stärkefaktor von zwischen etwa 0,13 mm bis etwa 0,3 mm oder mehr bevorzugt von etwa 0,15 mm bis etwa 0,25 mm aufweist, wie durch die hierin eingehend beschriebenen Stärke- und Stärkefaktorverfahren bestimmt.

**4.** Fluidmanagementschicht nach einem der vorstehenden Ansprüche, die ein Flächengewicht von zwischen etwa 45 g/m$^2$ bis etwa 70 g/m$^2$ aufweist, wie durch das hierin eingehend beschriebene Flächengewichtsverfahren bestimmt.

**5.** Fluidmanagementschicht nach einem der vorstehenden Ansprüche, wobei der Gewichtsprozentsatz von Absorptionsfasern zwischen 20 Prozent und etwa 30 Prozent liegt.

**6.** Fluidmanagementschicht nach einem der vorstehenden Ansprüche, wobei die elastischen Fasern eine lineare Dichte von etwa 4 dtex bis etwa 15 dtex, mehr bevorzugt von etwa 5 dtex bis etwa 12 dtex oder am meisten bevorzugt von etwa 6 dtex bis etwa 10 dtex umfassen, wie durch das hierin eingehend beschriebene Faser-Decitex-Verfahren bestimmt.

**7.** Fluidmanagementschicht nach einem der vorstehenden Ansprüche, wobei die Versteifungsfasern eine lineare Dichte von zwischen etwa 1,0 dtex bis etwa 6 dtex, mehr bevorzugt von etwa 1,5 dtex bis etwa 5 dtex oder am meisten bevorzugt von etwa 2,0 dtex bis etwa 4 dtex umfassen, wie durch das hierin eingehend beschriebene Faser-Decitex-Verfahren bestimmt.

**8.** Fluidmanagementschicht nach einem der Ansprüche 1 bis 5, wobei der Gewichtsprozentsatz der Absorptionsfasern zwischen 30 Prozent und etwa 60 Prozent liegt.

**9.** Fluidmanagementschicht nach Anspruch 8, wobei die elastischen Fasern eine lineare Dichte von etwa 5 dtex bis etwa 15 dtex, mehr bevorzugt von etwa 6 dtex bis etwa 12 dtex oder am meisten bevorzugt von etwa 7 dtex bis etwa 10 dtex umfassen, wie durch das hierin eingehend beschriebene Faser-Decitex-Verfahren bestimmt.

**10.** Fluidmanagementschicht nach Anspruch 8 und 9, wobei die Versteifungsfasern eine lineare Dichte von etwa 3 dtex bis etwa 6 dtex, mehr bevorzugt von etwa 4 dtex bis etwa 6 dtex umfassen, wie durch das hierin eingehend beschriebene Faser-Decitex-Verfahren bestimmt.

**11.** Einweg-Absorptionsartikel, umfassend eine Oberschicht, eine Unterschicht, einen Absorptionskern, der zwischen der Oberschicht und der Unterschicht angeordnet ist, der Einweg-Absorptionsartikel ferner umfassend die Fluidmanagementschicht nach einem der vorstehenden Ansprüche.

**12.** Einweg-Absorptionsartikel nach Anspruch 11, wobei die Fluidmanagementschicht zwischen der Oberschicht und dem Absorptionskern angeordnet ist.

**Revendications**

**1.** Couche de gestion de fluide comprenant un non-tissé cardé intégré ayant une masse surfacique comprise entre environ 40 grammes par mètre carré (g/m$^2$) et environ 75 g/m$^2$, telle que déterminée par la méthode de masse surfacique détaillée ici, et comprenant une pluralité de fibres absorbantes, une pluralité de fibres de raidissement et une pluralité de fibres élastiques, dans laquelle la couche de gestion de fluide a un facteur d'épaisseur d'au moins environ 0,13 tel que déterminé par les méthodes d'épaisseur et de facteur d'épaisseur détaillées ici ;

dans laquelle les fibres absorbantes sont présentes en une quantité comprise entre environ 10 pour cent en poids et environ 60 pour cent en poids, les fibres élastiques sont présentes en une quantité comprise entre environ 15 pour cent en poids et environ 70 pour cent en poids, et les fibres de raidissement sont présentes en une quantité comprise entre environ 25 pour cent en poids et environ 70 pour cent en poids telle que déterminée par la méthode d'analyse de composition de matériau détaillée ici ;
dans laquelle la couche de gestion de fluide est un non-tissé intégré par aiguilletage.

**2.** Couche de gestion de fluide selon la revendication 1, dans laquelle le non-tissé présente un temps d'acquisition de fluide inférieur à 10 secondes tel que mesuré par la méthode de temps de transpercement des liquides détaillée ici.

**3.** Couche de gestion de fluide selon l'une quelconque des revendications précédentes, dans laquelle le non-tissé a un facteur d'épaisseur allant d'environ 0,13 mm à environ 0,3 mm ou plus préférablement d'environ 0,15 mm à environ 0,25 mm tel que déterminé par les méthodes d'épaisseur et de facteurs d'épaisseur détaillées ici.

**4.** Couche de gestion de fluide selon l'une quelconque des revendications précédentes, ayant une masse surfacique comprise entre environ 45 g/m$^2$ et environ 70 g/m$^2$ telle que déterminée par la méthode de masse surfacique détaillée ici.

5. Couche de gestion de fluide selon l'une quelconque des revendications précédentes, dans laquelle le pourcentage en poids de fibres absorbantes est compris entre 20 pour cent et environ 30 pour cent.

6. Couche de gestion de fluide selon l'une quelconque des revendications précédentes, dans laquelle les fibres élastiques comprennent une masse linéique allant d'environ 4 dtex à environ 15 dtex, plus préférablement d'environ 5 dtex à environ 12 dtex, ou le plus préférablement d'environ 6 dtex à environ 10 dtex, telle que déterminée par la méthode de décitex de fibres détaillée ici.

7. Couche de gestion de fluide selon l'une quelconque des revendications précédentes, dans laquelle les fibres de raidissement comprennent une masse linéique allant d'environ 1,0 dtex à environ 6 dtex, plus préférablement d'environ 1,5 dtex à environ 5 dtex, ou le plus préférablement d'environ 2,0 dtex à environ 4 dtex, telle que déterminée par la méthode de décitex de fibres détaillée ici.

8. Couche de gestion de fluide selon l'une quelconque des revendications 1 à 5, dans laquelle le pourcentage en poids des fibres absorbantes est compris entre 30 pour cent et environ 60 pour cent.

9. Couche de gestion de fluide selon la revendication 8, dans laquelle les fibres élastiques comprennent une masse linéique allant d'environ 5 dtex à environ 15 dtex, plus préférablement d'environ 6 dtex à environ 12 dtex, ou le plus préférablement d'environ 7 dtex à environ 10 dtex tel que déterminé par la méthode de décitex de fibres détaillée ici.

10. Couche de gestion de fluide selon les revendications 8 et 9, dans laquelle les fibres de raidissement comprennent une masse linéique allant d'environ 3 dtex à environ 6 dtex, plus préférablement d'environ 4 dtex à environ 6 dtex, telle que déterminée par la méthode de décitex de fibres détaillée ici.

11. Article absorbant jetable comprenant une feuille de dessus, une feuille de fond, une âme absorbante disposée entre la feuille de dessus et la feuille de fond, l'article absorbant jetable comprenant en outre la couche de gestion de fluide selon l'une quelconque des revendications précédentes.

12. Article absorbant jetable selon la revendication 11, dans lequel la couche de gestion de fluide est disposée entre la feuille de dessus et l'âme absorbante.

FIG. 1A

FIG. 1B

FIG. 2

EP 4 324 444 B1

FIG. 3

FIG. 4

FIG. 5A

FIG. 5B

FIG. 5C

## FIG. 5D

| HV | HFW | mag ⊞ | WD | 11/20/2019 | | 500 μm |
|---|---|---|---|---|---|---|
| 5.00 kV | 1.38 mm | 150 x | 13.6 mm | 6:20:03 AM | ⋀ ANALYTICAL | Paper Analytical Micro NM |

FIG. 6A

FIG. 6B

FIG. 6C

FIG. 6D

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180098889 A
- US 20140343523 A
- US 6333108 B
- US 5634914 A, Wilkes
- US 5458835 A, Wilkes
- US 7767598 B, Schneider
- US 3929135 A, Thompson
- US 4324246 A, Mullane
- US 4342314 A, Radel
- US 4463045 A, Ahr
- US 5006394 A, Baird
- US 4609518 A,  Curro
- US 4629643 A, Curro
- US 9700463 B
- US 9849602 B
- US 5885265 A, Osborn, III.
- US 6462251 B, Cimini
- US 6623464 B, Bewick-Sonntag
- US 6664439 B, Arndt
- US 3881489 A
- US 4341216 A
- US 4713068 A
- US 4818600 A
- EP 203821 A
- EP 710471 A
- EP 710472 A
- EP 793952 A
- GB 2184389 A
- GB 2184390 A
- GB 2184391 A
- US 4591523 A
- US 3989867 A
- US 3156242 A
- WO 9724097 A
- US 6436508 B, Ciammaichella
- US 5599335 A, Goldman
- EP 1447066 A, Busam
- WO 9511652 A, Tanzer
- US 20080312622 A1,  Hundorf
- WO 2012052172 A, Van Malderen
- US 4610678 A,  Weisman
- US 4673402 A,  Weisman
- US 4888231 A
- US 4834735 A, Alemany
- US 5234423 A,  Alemany
- US 5147345 A
- US 20180098893
- US 20180098891
- US 20100228209 A1
- US 8466336 B, Carlucci
- US 9693910 B, Carlucci
- US 4695278 A
- US 4704115 A
- US 4795454 A
- US 4909803 A
- US 20090312730
- US 20180098891 A

**Non-patent literature cited in the description**

- *CHEMICAL ABSTRACTS*, 109-99-9